(19) Europäisches Patentamt
European Patent Office
Office européen des brevets

(11) EP 1 268 479 B1

(12) **EUROPEAN PATENT SPECIFICATION**

(45) Date of publication and mention of the grant of the patent:
**28.01.2004 Bulletin 2004/05**

(21) Application number: **01928331.6**

(22) Date of filing: **29.03.2001**

(51) Int Cl.$^7$: **C07D 471/04**, A61K 31/4353, A61P 43/00
// (C07D471/04, 221:00), C07D209:00,(C07D471/04, 231:00), C07D221:00

(86) International application number:
**PCT/US2001/010049**

(87) International publication number:
**WO 2001/074814 (11.10.2001 Gazette 2001/41)**

(54) **PHENYL-SUBSTITUTED INDOLIZINE DERIVATIVES AND THEIR USE AS HISTAMINE H3 LIGANDS**

PHENYL-SUBSTITUIERTE INDOLIZINDERIVATE UND DEREN VERWENDUNG ALS HISTAMIN H3 LIGANDEN

DERIVES D'INDOLIZINE SUBSTITUES PHENYLE ET LEURS UTILISATIONS EN TANT QUE LIGANDS H3 DE L'HISTAMINE

(84) Designated Contracting States:
**AT BE CH CY DE DK ES FI FR GB GR IE IT LI LU MC NL PT SE TR**

(30) Priority: **31.03.2000 US 194071 P**
**28.02.2001 US 272137 P**

(43) Date of publication of application:
**02.01.2003 Bulletin 2003/01**

(73) Proprietor: **Ortho-McNeil Pharmaceutical, Inc.
Raritan, NJ 08869-0602 (US)**

(72) Inventors:
- **CHAI, Wenying
San Diego, CA 92126 (US)**
- **KWOK, Annette K.
San Diego, CA 92126 (US)**
- **LI, Xiaobing
New Jersey 08822 (US)**
- **RUDOLPH, Dale A.
San Diego, CA 92126 (US)**

(74) Representative: **Fisher, Adrian John et al
CARPMAELS & RANSFORD
43 Bloomsbury Square
London WC1A 2RA (GB)**

(56) References cited:
**EP-A- 0 978 512**

EP 1 268 479 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The invention relates to pharmaceutically-active fused heterobicyclic compounds and methods of using them to treat or prevent disorders and conditions, such as those mediated by the histamine $H_3$ receptor.

## Background

The histamine $H_3$ receptor is located as a presynaptic autoreceptor in the central nervous system and as a presynaptic heteroreceptor on serotonergic, noradrenergic, dopaminergic, and cholinergic neurons. The histamine $H_3$ receptor is also located peripherally in tissues such as vascular smooth muscle cells.

Proposed uses of histamine $H_3$ antagonists include the treatment or prevention of dementia, Alzheimer's disease (Panula et al. *Abstr. Society Neuroscience,* 1995, 21:1977), epilepsy (Yokoyama et al. *Eur. J. Pharmacol*., 1993, 234:129), sleep/wake disorders (*Lin et al., Br. Res.,* 1990, 523, 325; Monti *et al., Eur. J. Pharmacol*., 1991, 205, 283) including narcolepsy, insomnia, and jet lag, eating disorders (Machidori et al. *Brain Research,* 1992, 590:180), motion sickness, vertigo, attention deficit hyperactivity disorder, learning and memory disorders (Barnes et al. *Abstr. Society Neuroscience*, 1993, 19:1813), schizophrenia (Schlicker et al. *Naunyn Schmiedeberg's Arch. Pharmacol*., 1996, 353:325), and sequelae associated with post-ischemic reperfusion and hypertension (Imamura *et al., J. Pharmacol. Expt. Ther.,* 1994, 271, 1259). $H_3$ antagonists are also useful to treat or prevent neurogenic inflammation such as migraine (McLeod *et al., Abstr. Society Neuroscience,* 1996, 22, 2010), asthma (Ichinose *et al., Eur. J. Pharmacol*., 989, 174, 49), obesity, allergic rhinitis, substance abuse, bipolar disorders, manic disorders, and depression. Histamine $H_3$ antagonists alone or in combination with a histamine $H_1$ antagonist are believed to be useful in the treatment of upper airway allergic response or allergic rhinitis (see, e.g., US Patent Nos. 5217986, 5352707, and 5869479).

As noted, the prior art related to histamine $H_3$ ligands was comprehensively reviewed recently *("The Histamine $H_3$ Receptor-A Target for New Drugs",* Leurs, R., and Timmerman, H., (Editors), Elsevier, 1998). Within this reference the medicinal chemistry of histamine $H_3$ agonists and antagonists was reviewed (see Krause et al. and Phillips et al., respectively). Thus the importance of an imidazole moiety containing only a single substitution in the 4 position was noted together with the deleterious effects of additional substitution on activity. Particularly methylation of the imidazole ring at any of the remaining unsubstituted positions was reported to strongly decrease activity.

More recently several publications have described histamine $H_3$ ligands that do not contain an imidazole moiety. Examples include Ganellin et al *Arch. Pharm. (Weinheim, Ger.)* 1998, 331, 395; Walczynski et *al Arch. Pharm. (Weinheim,Ger.)* 1999, 332, 389; Walczynski et al Farmaco 1999, 684; Linney et al *J. Med. Chem*. 2000, 2362; U.S. Patent 5,352,707; PCT Application WO99/42458, published August 26, 1999; and European Patent Application 0978512, published on February 9, 2000.

## Summary of the Invention

The invention features phenyl-substituted indolizines and tetrahydroindolizine compounds, methods of making them, and methods of using them. One aspect of the invention provides compounds of the following formula (I)(C):

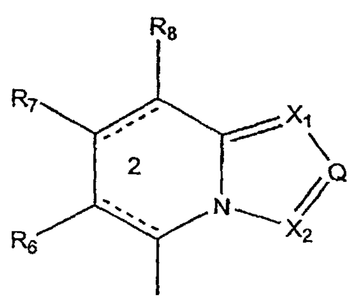

wherein

- the dashed lines are both present to form two carbon-carbon double bonds; or are both absent;
- $X_1$ is $CR_1$, wherein $R_1$ is H, $C_{1-6}$ alkyl, phenyl, cyano, or phenyl($C_{1-6}$ alkyl);
- one of Q and $X_2$ is C-Ar and the other is $CR_3$ or N, where $R_3$ is H or $C_{1-6}$ alkyl;
- Ar is:

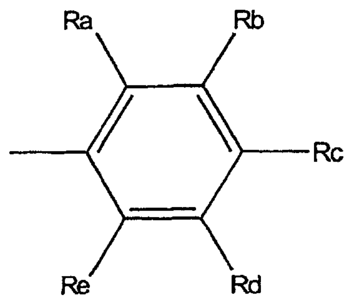

each of $R_5$, $R_6$, $R_7$, and $R_8$ are independently selected from H, $C_{1-3}$ alkyl, halo, and $C_{1-3}$ alkoxy;

one of $R_a$, $R_b$, $R_c$, $R_d$, and $R_e$ is WZ, and the others are independently selected from H, $C_{1-6}$ alkyl, halo, and $C_{1-6}$ alkoxy;

W is -O-, $C_{1-6}$ alkoxy, or $C_{1-6}$ alkylamino;

Z is $C_{2-8}$ heterocyclic radical, optionally including in the ring up to 3 additional heteroatoms or moieties independently selected from O, N, NH, S, SO, and $SO_2$ with at least one basic N; or Z is $NR_{11}R_{12}$ where each of $R_{11}$ and $R_{12}$ is independently selected from H, $C_{1-6}$ alkyl, phenyl, benzyl, $C_{3-8}$ cycloalkyl, and $C_{2-5}$ heterocyclic radical; or $NR_{11}R_{12}$ taken together is $C_{6-8}$ cycloalkylimino radical;

each of the above hydrocarbyl, heteroalkyl, or heterocyclic groups being optionally substituted with between 1 and 3 substituents selected from

$C_{1-3}$ alkyl, halo, hydroxy, phenyl, and phenyl($C_{1-3}$ alkyl); and wherein each of the above heterocyclic groups may be attached to the rest of the molecule by a carbon atom or a heteroatom;

or a pharmaceutically acceptable salt, amide, ester, or hydrate thereof.

According to another aspect of the invention, the disclosed compounds and certain other compounds, are useful for the treatment and/or prevention of diseases and conditions mediated by the histamine 3 ($H_3$) receptor

A third aspect of the invention features methods of making the disclosed compounds.

Additional features of the invention are disclosed in the following description and examples, and in the appended claims.

## Detailed Description

The invention features pharmaceutically active phenyl-substituted indolizines and tetrahydroindolizines and methods of making and using them. The description is organized as follows:

A. Terms
B. Compounds
C. Synthetic Methods
D. Uses (including dosages, formulations, and related compounds)
E. Synthetic Examples
F. Biological Examples
G. Other Embodiments
H. Claims

### A. Terms

The following terms are defined below and by their usage throughout this disclosure.

"Alkyl" includes straight chain and branched hydrocarbons with at least one hydrogen removed to form a radical group. Alkyl groups include methyl, ethyl, propyl, isopropyl, butyl, isobutyl, t-butyl, 1-methylpropyl, pentyl, isopentyl, sec-pentyl, hexyl, heptyl, octyl, and so on. Alkyl does not include cycloalkyl.

"Alkenyl" includes straight chain and branched hydrocarbon radicals as above with at least one carbon-carbon double bond ($sp^2$). Alkenyls include ethenyl (or vinyl), prop-1-enyl, prop-2-enyl (or allyl), isopropenyl (or 1-methylvinyl), but-1-enyl, but-2-enyl, butadienyls, pentenyls, hexa-2,4-dienyl, and so on. Hydrocarbon radicals having a mixture of double bonds and triple bonds, such as 2-penten-4-ynyl, are grouped as alkynyls herein. Alkenyl does not include cycloalkenyl.

"Alkynyl" include straight chain and branched hydrocarbon radicals as above with at least one carbon-carbon

triple bond (sp). Alkynyls include ethynyl, propynyls, butynyls, and pentynyls. Hydrocarbon radicals having a mixture of double bonds and triple bonds, such as 2-penten-4-ynyl, are grouped as alkynyls herein. Alkynyl does not include cycloalkynyl.

"Alkoxy" includes a straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing the terminal oxygen atom of alkoxy with, respectively, NH (or NR), S, and $SO_2$. Heteroalkyl includes alkoxy, aminoalkyl, thioalkyl, and so on.

"Aryl" includes phenyl, naphthyl, biphenylyl, and so on.

"Cycloalkyl" includes cyclopropyl, cyclobutyl, cyclopentyl, cyclohexyl, cycloheptyl, cyclooctyl, and so on.

"Cycloalkenyl" includes cyclobutenyl, cyclobutadienyl, cyclopentenyl, cyclopentadienyl, cyclohexenyl, cyclohexadienyl, cyclohexatrienyl (phenyl), cycloheptenyl, and so on. "Cycloalkynyl" includes the analogous rings with one or more triple bonds.

"Heterocyclic radicals" include aromatic and nonaromatic rings having carbon atoms and at least one heteroatom (O, S, N) or heteroatom moiety ($SO_2$, CO, CONH, COO) in the ring. Unless otherwise indicated, a heterocyclic radical may have a valence connecting it to the rest of the molecule through a carbon atom, such as 3-furyl or 2-imidazolyl, or through a heteroatom, such as N-piperidyl or 1-pyrazolyl. Examples of heterocyclic radicals include thiazoylyl, furyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imdazolidinyl, imidazolinyl, pyrazolidinyl, pyrazolinyl, piperidyl, piperazinyl, indolinyl, and morpholinyl. For example, preferred heterocyclic radicals for Z include morpholinyl, piperazinyl, pyrrolidinyl, pyridyl, cyclohexylimino, cycloheptylimino,and more preferably, piperidyl.

"halo" includes fluoro, chloro, bromo, and iodo, and preferably fluoro or chloro.

"patient" or "subject" includes mammals such as humans and animals (dogs, cats, horses, rats, rabbits, mice, non-human primates) in need of observation, experiment, treatment or prevention in connection with the relevant disease or condition. Preferably, the patient is a human.

"composition" includes a product comprising the specified ingredients in the specified amounts as well as any product which results directly or indirectly from combinations of the specified ingredients in the specified amounts.

Concerning the various radicals in this disclosure and in the claims, two general remarks are made. The first remark concerns valency. As with all hydrocarbon radicals, whether saturated, unsaturated or aromatic, and whether or not cyclic, straight chain, or branched, and also similarly with all heterocyclic radicals, each radical includes substituted radicals of that type and monovalent, bivalent, and multivalent radicals as indicated by the context of the claims. The context will indicate that the substituent is an alkylene or hydrocarbon radical with at least two hydrogen atoms removed (bivalent) or more hydrogen atoms removed (multivalent). An example of a bivalent radical linking two parts of the molecule is W in formula (I)(C) which links Z with the phenyl group Ph (-Ph-W-Z). Subject to the claims, the bivalent group W can be an aminoalkyl group ($-Ph-NH-CH_2CH_2CH_2-Z$), an alkoxy group ($-Ph-O-CH_2CH_2CH_2-Z$), an "oxa" (-Ar-O-Z), or a covalent bond (-Ph-Z), and so on.

Second, radicals or structure fragments as defined herein are understood to include substituted radicals or structure fragments. Using "alkyl" as an example, "alkyl" should be understood to include substituted alkyl having one or more substitutions, such as between 1 and 5, 1 and 3, or 2 and 4 substituents. The substituents may be the same (dihydroxy, dimethyl), similar (chlorofluoro), or different (chlorobenzyl- or aminomethyl-substituted). Examples of substituted alkyl include haloalkyl (such as fluoromethyl, chloromethyl, difluoromethyl, perchloromethyl, 2-bromoethyl, perfluoromethyl, and 3-iodocyclopentyl), hydroxyalkyl, aminoalkyl, nitroalkyl, alkylalkyl, and so on.

Preferred substitutions for Ar include methyl, methoxy, fluoromethyl, difluoromethyl, perfluoromethyl (trifluoromethyl), 1-fluoroethyl, 2-fluoroethyl, ethoxy, fluoro, chloro, and bromo, and particularly methyl, fluoromethyl, perfluoro, methoxy, and fluoro.

Examples of other substituted radicals or fragments include 1-methyl-2-pyrrolidino, 4-(piperidyl)-piperidyl, [4-(N-benzyl)piperidyl]-amino, 4-fluorobenzylamino, beta-hydroxyethoxy, beta-hydroxy-propoxy, 2-oxo-pyrrolidino, 4-(1-methyl-4-piperidinyl), 4-(5-methyl-thiazoyl), 4-chlorobenzyl, 4-fluorobenzyl, and 4-methylbenzyl.

B. Compounds

One aspect of the invention features compounds of formula (I)(C) as described in the Summary section above.

Preferred compounds of formula (I)(C) include those compounds wherein: (a) one of $R_a$, $R_b$, $R_c$, $R_d$, and $R_e$ is WZ, and the others are independently selected from H, $C_{1-3}$ alkyl, fluoro, chloro, and $C_{1-3}$ alkoxy; (b) between one and two of $R_1$, $R_3$, $R_5$, $R_6$, $R_7$, and $R_8$ independently comprise methyl, ethyl, $-CH_2-$, $-CH_2-CH_2-$, or phenyl (for example, methyl, ethyl, fluoromethyl, chlorophenyl, phenyl, hydroxyethyl, phenethyl, hydroxybenzyl, or hydroxyphenyl); (c) one of $R_1$ and $R_3$ comprises methyl, ethyl, $-CH_2-$, $-CH_2-CH_2-$, or phenyl; (d) both dashed lines are absent, forming a tetrahydroindolizine skeleton; (e) $X_2$ is $CR_3$; (f) both dashed lines are present, forming two carbon-carbon double bonds,

thereby forming a indolizine skeleton; (g) Q is C-Ar and $X_2$ is $CR_3$ or N, where $R_3$ is H or $C_{1-4}$ alkyl; (h) at least two of the following apply: Z is N-piperidyl; W comprises propoxy or (N-methyl)propylamino; and three of $R_a$, $R_b$, $R_d$, and $R_e$ are each H; (i) WZ is 3-(N-piperidyl)propoxy or 3-(N-piperidyl)-(N-methyl)propylamino; or combinations of the above.

Additional preferred compounds include those wherein: (k) $R_1$ is H, methyl, ethyl, propyl, butyl, phenyl, benzyl, or phenethyl; $R_3$ is H or $C_{1-2}$ alkyl; each of $R_5$, $R_6$, $R_7$, $R_8$ and $R_9$ is independently H or methyl; and one of $R_a$, $R_b$, $R_c$ and $R_d$ is WZ; and the remaining three are each H; W is $C_{2-4}$ alkoxy or $C_{2-4}$ alkylamino; and Z is $C_{2-8}$ heterocyclic radical, optionally including in the ring up to 3 additional heteroatoms or moieties independently selected from O, N, NH, S, SO, and $SO_2$ with at least one basic N; or Z is $NR_{11}R_{12}$ where each of $R_{11}$ and $R_{12}$ is independently selected from H, $C_{1-6}$ alkyl, phenyl, benzyl, $C_{3-8}$ cycloalkyl, and $C_{2-5}$ heterocyclic radical; or $NR_{11}R_{12}$ taken together is $C_{6-8}$ cycloalkylimino radical.

Examples of more preferred compounds include: (i) those selected from 3-Methyl-2-(4-piperidinylpropoxyphenyl)indolizine; 1-Methyl-2-(2-methyl-4-(3-piperidinylpropoxyphenyl))indolizine; 1-Phenyl-2-[4-(3-piperidinylpropoxy) phenyl]indolizine; 1-Phenethyl-2-[4-(3-piperidinylpropoxy)phenyl]indolizine; 1-Ethyl-2-[4-(3-piperidinylpropoxy)phenyl]indolizine; 1-Methyl-2-[4-(3-piperidinylpropoxy)-phenyl]-indolizine; 2-(3-Piperidinopropoxyphenyl)indolizine and 3-Ethyl-2-[4-(3-piperidinylpropoxy)phenyl]indolizine; [5-Methyl-2-(4-piperidinylpropoxyphenyl)indolizine; and 8-Methyl-2-[4-(3-piperidinylpropoxy)-phenyl]-indolizine ; and (ii) 2-(4-Piperidylpropoxyphenyl)-7a-hydropyrazolo[1,5-2]pyridine.

Other examples of compounds, and methods of making them, are provided in the next section.

C. Synthetic Methods

The invention provides methods of making the disclosed compounds according to traditional organic synthetic methods as well as matrix or combinatorial synthetic methods. Schemes 1 through 3 describe suggested synthetic routes.

Using these Schemes, the guidelines below, and the examples in section E, a person of skill in the art may develop analogous or similar methods for a given compound.

Examples of the described synthetic routes includes Synthetic Examples 1 through 14. Compounds analogous to the target compounds of these examples can be, and in many cases, have been, made according to similar routes. The disclosed compounds are useful in basic research and as pharmaceutical agents as described in the next section.

Compounds of formula I may be prepared according to the processes outlined in Schemes 1 through 3. It should noted that throughout the Schemes the position of substitution is indicated by defining substituent Rc in formula I. However it will be recognised by one skilled in the art that the substituent may be located at Ra, Rb, Rd or Re and that position Rc is chosen for illustrative purposes only.

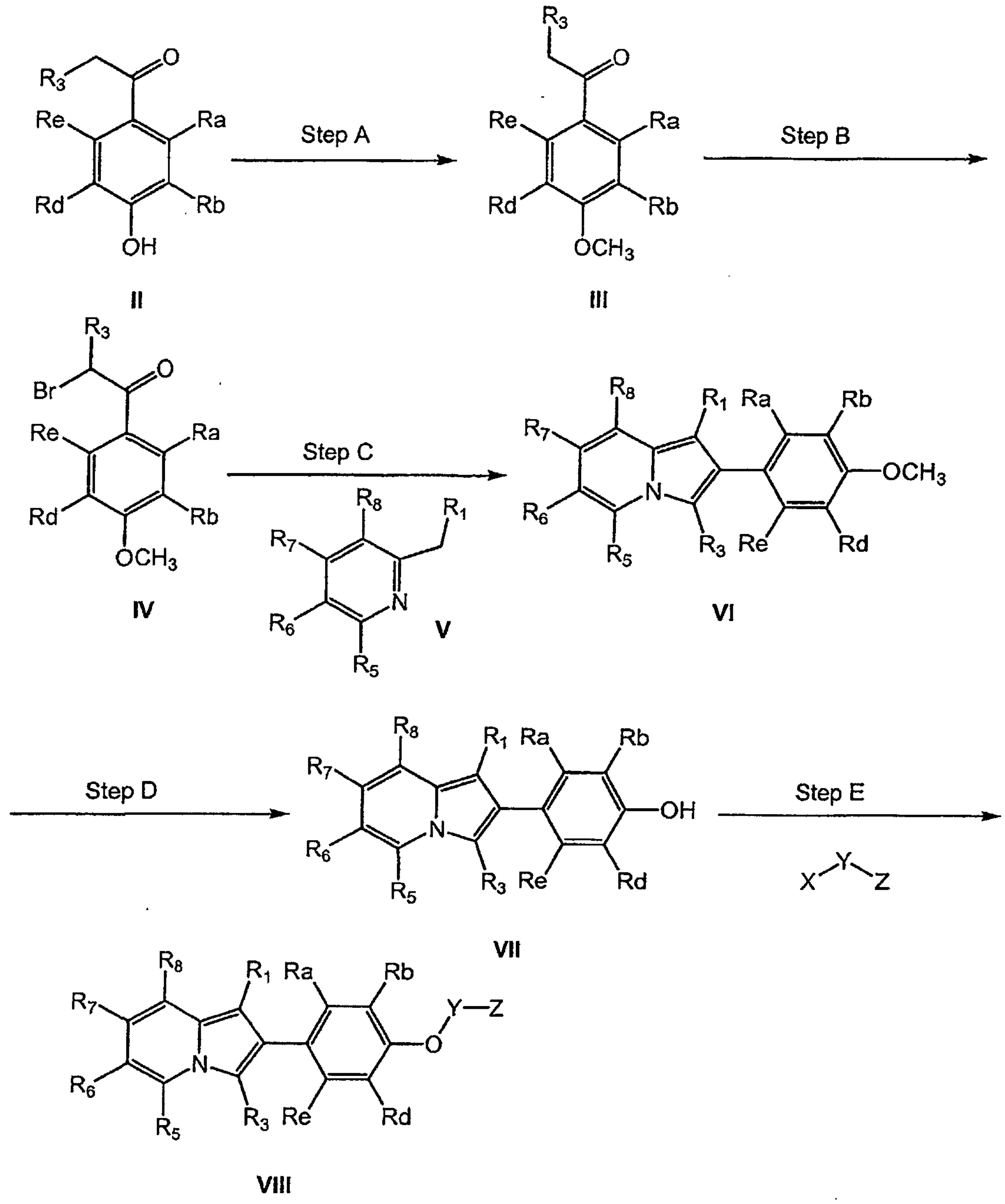


SCHEME 1

Compounds of formula VIII, wherein the substituents are as defined in formula I, may be prepared according to the process outlined in Scheme I. Specifically a compound of formula II is converted to a compound of formula III in Step A upon treatment with methyl iodide, dimethylsulfate, in the presence of a base, for example potassium carbonate, lithium carbonate, or sodium hydroxide in a solvent such as DMF, acetone, THF, or dichloromethane. In a preferred embodiment the compound of formula II is treated with methyl iodide in acetone in the presence of potassium carbonate to afford a compound of formula III. In Step B a compound of formula III is converted to a compound of formula IV upon treatment with bromine in a solvent such as ether, chloroform, dichloromethane, or carbontetrachloride. A compound of formula VI may be obtained in Step C by reacting a compound of formula IV with a compound of formula V in a solvent such as acetone or 2-butanone at elevated temperature, preferably at the boiling point of the selected solvent, to give an intermediate salt that may be isolated by filtration. The intermediate may be dissolved in water, treated with a base, for example potassium carbonate, sodium carbonate, sodium hydroxide or the like and heated at 50° C to 100° C. In a preferred embodiment the intermediate salt is dissolved in water and heated at 80° C to afford

a compound of formula VI.

A compound of formula VII may be obtained from a compound of formula VI according to the procedure of Step D, whereupon a compound of formula VI is treated with trimethylsilyliodide in chloroform, sodium ethanthiolate in DMF, sodium cyanide in DMSO, boron tribromide in dichloromethane, or hydrogen bromide in acetic acid. In preferred embodiments a compound of formula VI is treated with sodium ethanthiolate in DMF at 60° C to 100° C, preferably at 100° C or treated with 48% hydrogen bromide in acetic acid at 100° C to afford a compound of formula VII.

A compound of formula VIII may be obtained, in Step E, by reacting an appropriately substituted compound of formula X-Y-Z; where Y is defined as $C_{1-6}$ alkyl, $C_{2-6}$ alkynyl, $C_{2-6}$ alkenyl or $C_{1-6}$ alkoxy, Z is as defined and X is selected from the group consisting of Cl, Br, I, mesylate and tosylate. This transformation is effected in the presence of a base, for example potassium carbonate, sodium hydroxide, sodium methoxide, or triethylamine in a solvent, for example ethanol, methanol, acetone, dichloromethane, DMF, or THF. In a preferred embodiment X is chlorine, the base is sodium methoxide and the solvent DMF at 80° C.

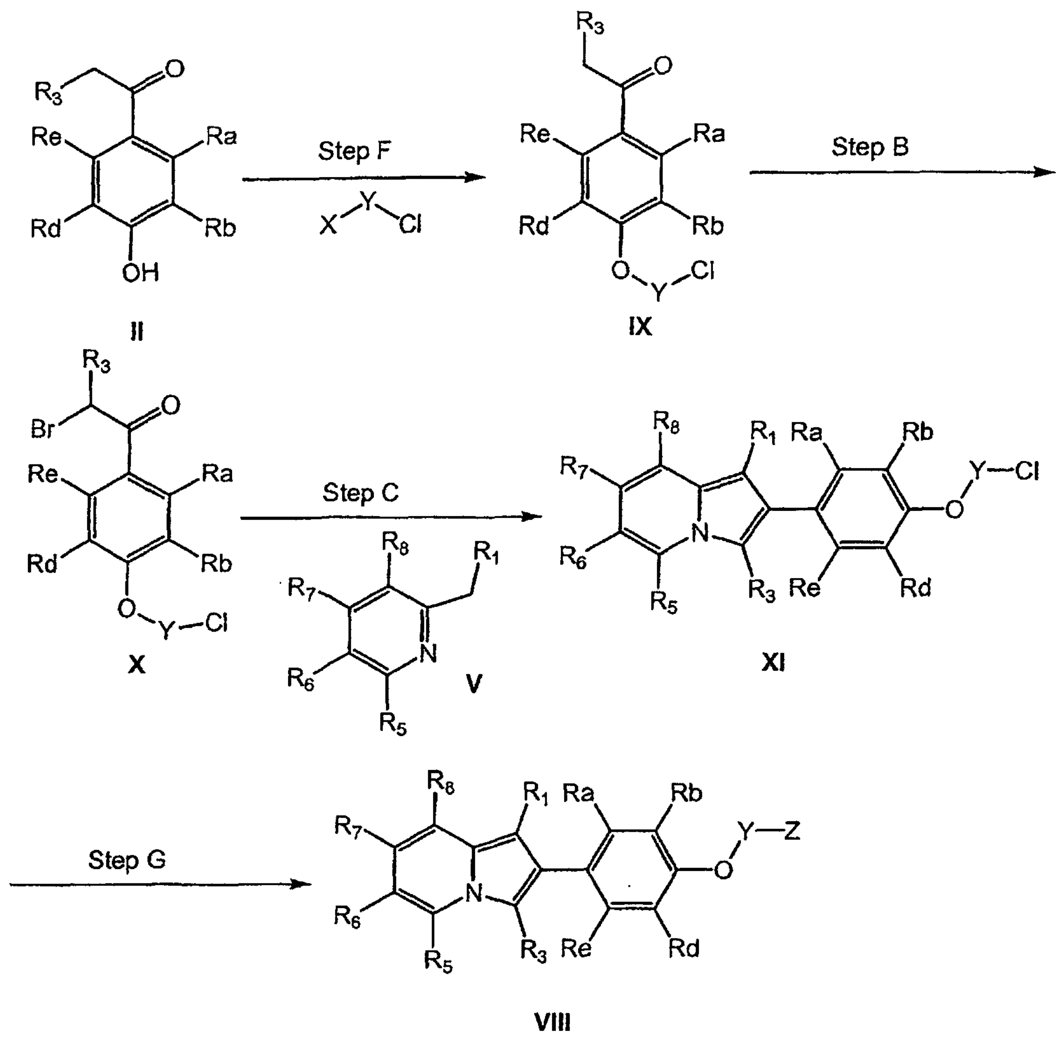

II
IX
X
V
XI
VIII

SCHEME 2

Compounds of formula VIII may be prepared according to the procedures shown in Scheme 2. In Step F a compound of formula II is reacted with a suitably substituted compound of formula X-Y-Cl where Y is defined and X is selected from the group consisting of Br, I, mesylate and tosylate such that under the reaction conditions a compound of formula IX is obtained. This transformation is effected in the presence of a base, for example potassium carbonate, sodium hydroxide, or triethylamine, in a solvent, for example ethanol, methanol, acetone, dichloromethane, DMF, or THF. Preferred conditions use potassium carbonate in acetone.

A compound of formula XI may be obtained by reacting a compound of formula X with a compound of formula V according to Step C of Scheme 1. A compound of formula VIII may be obtained in Step G by reacting a compound of formula XI with an amine at elevated temperature, preferably neat amine at a temperature from 50° C to the boiling point of the amine, more preferably at about 80° C to 100° C. Alternatively the compound of formula XI may be treated with an amine in the presence of a base, for example potassium carbonate in a solvent, for example acetone at elevated temperature, for example at about 55° C.

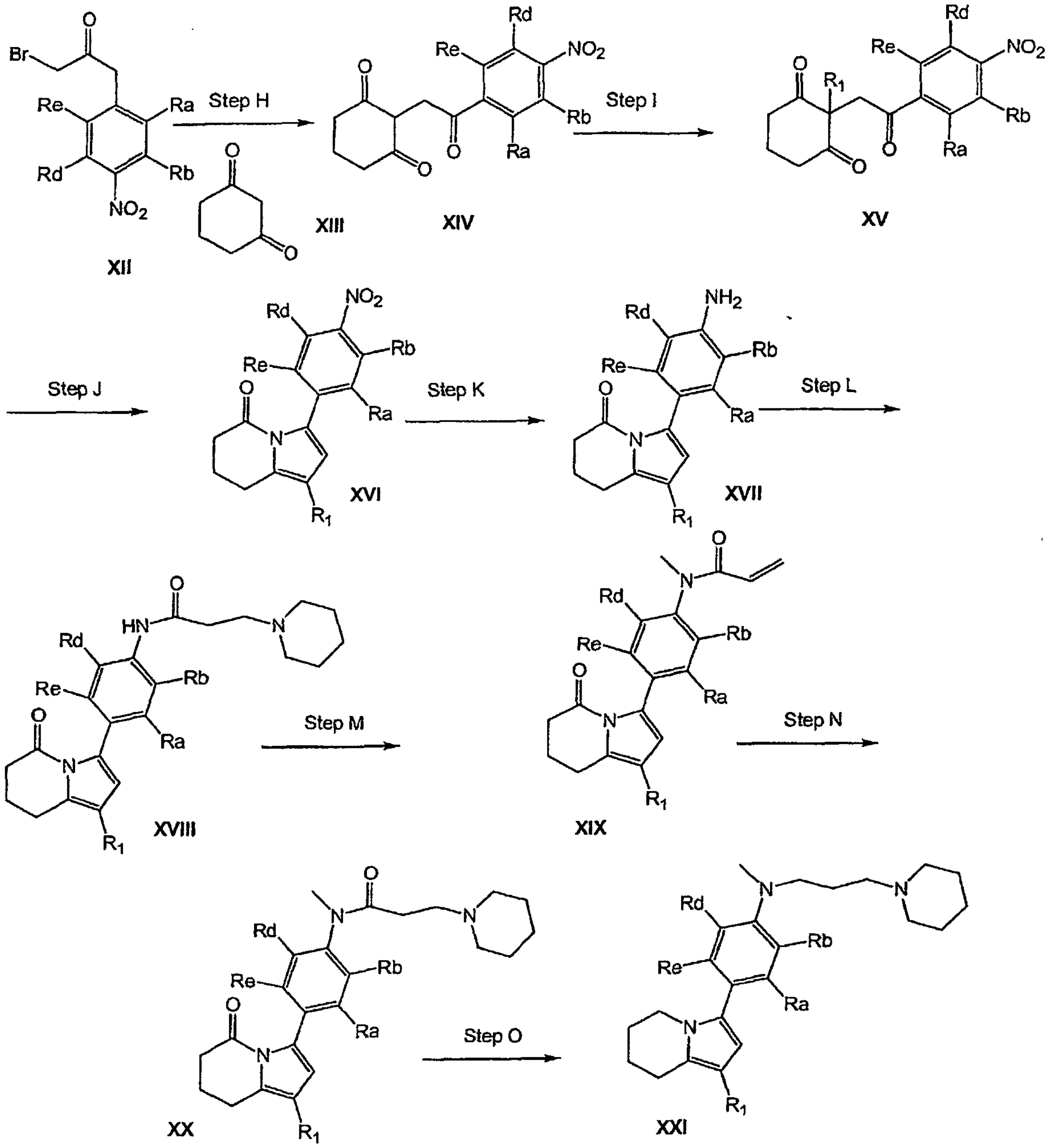


SCHEME 3

A compound of formula XXI may be prepared according to the processes shown in Scheme 3. Thus a compound of formula XIV may be prepared in Step H by reacting a compound of formula XII with a compound of formula XIII. In a preferred embodiment the compound of formula XII is reacted with 1,3-cyclohexandione in the presence of a base, for example potassium carbonate, sodium carbonate, or sodium hydroxide in a solvent, for example, chloroform, dichloromethane, or toluene. In a more preferred embodiment the base is potassium carbonate and the solvent is chloroform.

A compound of formula XV may be prepared by reacting a compound of formula XIV with an alkylating agent for example an alkyl iodide in the presence of a base, for example DBU, DABCO, or lithium diisopropylamide. In a preferred embodiment the alkyl iodide is methyl iodide and the base is DBU in the solvent DMF. A compound of formula XV may be converted to a compound of formula XVI in Step J, whereupon a compound of formula XV is treated with ammonium acetate in acetic acid at elevated temperature, for example about 80° C to 200° C and preferably at about 120° C, to effect rearrangement and cyclization to give a compound of formula XVI. A compound of formula XVI may be reduced to a compound of formula XVII in Step K. Thus the nitro functionality may be reduced to the corresponding amine to give compounds of formula XVII. Reduction may be effected via hydrogen gas over a catalyst, for example palladium on carbon, platinum oxide, or Raney nickel in a solvent, for example methanol, ethanol or the like. Reduction may also be effected via transfer hydrogenation techniques, for example using cyclohexadiene as a source of hydrogen. Reduction may also be effected using zinc, tin chloride or iron in the presence of an acid, for example hydrochloric acid. A preferred method of reduction is the use of tin chloride in ethanol at elevated temperature, preferably at about 80° C.

A compound of formula XVII may be converted to a compound of formula XVIII in Step L by reacting a compound of formula XVII with 3-piperidinepropionic acid using an active ester procedure. In a preferred embodiment a compound of formula XVII in dichloromethane is reacted with 3-piperidinepropionic acid in the presence of 1-hydroxybenzotriazole and 1-ethyl-3-(3'-dimethylaminopropyl)carbodiimide hydrochloride in the presence of N, N-dimethylaminopyridine to give a compound of formula XVIII. In Step M a compound of formula XVIII may be treated with an alkyl iodide in the presence of a base. In a preferred embodiment the alkyl iodide is methyl iodide and the base is lithium hexamethyldisilazane in hexane and the solvent DMF. It would be recognized by one skilled in the art that the products of Step M can include compounds of both formula XIX and XX.

A compound of formula XIX may be converted to a compound of formula XX upon reaction with piperidine at elevated temperature according to Step N. A preferred embodiment involves the treatment of a compound of formula XIX with piperidine in toluene at 80° C to afford a compound of formula XX. A compound of formula XXI may be obtained from a compound of formula XX in Step O. Thus a compound of formula XX may be may be reduced to give a compound of formula XXI. Suitable reducing agents include lithium aluminum hydride, alane, sodium borohydride in the presence of acid and borane. A preferred method for the reduction of XX to XXI is borane in THF at 60° C.

D. Uses

According to the invention, the disclosed compounds and compositions are useful for the amelioration of symptoms associated with, the treatment of, and/or the prevention of, the following conditions and diseases, or symptoms associated with them: dementia, Alzheimer's disease, narcolepsy, eating disorders, motion sickness, vertigo, attention deficit hyperactivity disorder, leaming and memory disorders, schizophrenia, mild cognitive impairment, upper airway allergic response (allergic rhinitis), insomnia, jet lag, obesity, asthma, neurogenic inflammation, substance abuse, bipolar disorders, manic disorders, and depression. The invention also features pharmaceutical compositions, which include, without limitation, one or more of the disclosed compounds, and a pharmaceutically acceptable carrier or excipient.

1. Dosages

Those skilled in the art will be able to determine, according to known methods, the appropriate dosage for a patient, taking into account factors such as age, weight, general health, the type of symptoms requiring treatment, and the use of other medications. An effective amount means that amount of pharmaceutical reagent (such as a prodrug, metabolic precursor, or active compound) that elicits the biological or medical response desired. In general, a therapeutically effective amount will be between 0.01 and 1000 mg/kg per day, preferably between 0.01 and 250 mg/kg body weight, and daily dosages will be between 0.50 and 5000 mg for an adult subject of normal weight. Capsules, tablets or other formulations (such as liquids and film-coated tablets) may be of between 0.20 and 100 mg, such as 0.20, 0.50, 1.0, 2.0, 3.0, and 10 mg can be administered according to the disclosed methods.

2. Formulations

Dosage unit forms include tablets, capsules, pills, powders, granules, aqueous and nonaqueous oral solutions and suspensions, and parenteral solutions packaged in containers adapted for subdivision into individual doses. Dosage unit forms can also be adapted for various methods of administration, including controlled release formulations, such as subcutaneous implants. Administration methods include oral, rectal, parenteral (intravenous, intramuscular, subcutaneous), intracisternal, intravaginal, intraperitoneal, intravesical, local (drops, powders, ointments, gels or cream), and by inhalation (a buccal or nasal spray) as appropriate depending on the overall health and condition of

the patient as determined by a physician or veterinary doctor.

Parenteral formulations include pharmaceutically acceptable aqueous or nonaqueous solutions, dispersion, suspensions, emulsions, and sterile powders for the preparation thereof. Examples of carriers include water, ethanol, polyols (propylene glycol, polyethylene glycol), vegetable oils, and injectable organic esters such as ethyl oleate. Fluidity can be maintained by the use of a coating such as lecithin, a surfactant, or maintaining appropriate particle size. Carriers for solid dosage forms include (a) fillers or extenders, (b) binders, (c) humectants, (d) disintegrating agents, (e) solution retarders, (f) absorption accelerators, (g) adsorbants, (h) lubricants, (i) buffering agents, and (j) propellants.

Compositions may also contain adjuvants such as preserving, wetting, emulsifying, and dispensing agents; antimicrobial agents such as parabens, chlorobutanol, phenol, and sorbic acid; isotonic agents such as a sugar or sodium chloride; absorption-prolonging agents such as aluminum monostearate and gelatin; and absorption-enhancing agents.

3. Combination Therapy

The present invention also provides compositions and methods useful for the treatment of disorders or conditions modulated, preferably antagonized, by the histamine $H_3$ receptor in combination with compounds that modulate other receptors including, but not limited to, histamine $H_1$ and histamine $H_2$ receptors. The present invention includes compounds and compositions useful in methods of combination therapy for the treatment of diseases or conditions modulated by the histamine $H_3$ receptor in combination with compounds that are selective serotonin re-uptake inhibitors (SSRIs), such as PROZAC™, or are selective norepinephrine uptake inhibitors. Such combination methods include (a) administering the two or more pharmaceutical agents separately formulated and at separate times, and (b) administering the two or more agents simultaneously in a single formulation or in separate formulations administered more or less at the same time. For example, one aspect is a method of treatment comprising administering at least one histamine $H_3$ receptor modulating compound disclosed herein and administering at least one compound selected from a histamine $H_1$ receptor modulating compound, a histamine $H_2$ receptor modulating compound, a selective serotonin reuptake inhibitor (such as PROZAC™), or a selective norepinephrine uptake inhibiting compound.

4. Related Compounds

The invention provides the disclosed compounds and closely related, pharmaceutically acceptable forms of the disclosed compounds, such as salts, esters, amides, acids, hydrates or solvated forms thereof; masked or protected forms; and racemic mixtures, or enantiomerically or optically pure forms.

Pharmaceutically acceptable salts, esters, and amides include carboxylate salts (e.g., $C_{1-8}$ alkyl, cycloalkyl, aryl, heteroaryl, or non-aromatic heterocyclic) amino acid addition salts, esters, and amides which are within a reasonable benefit/risk ratio, pharmacologically effective and suitable for contact with the tissues of patients without undue toxicity, irritation, or allergic response. Representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. These may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine. See example, S.M. Berge, et al., "Pharmaceutical Salts," J. Pharm. Sci., 1977, 66:1-19 which is incorporated herein by reference. Representative pharmaceutically acceptable amides of the invention include those derived from ammonia, primary $C_{1-6}$ alkyl amines and secondary di ($C_{1-6}$ alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, $C_{1-3}$ alkyl primary amines, and di ($C_{1-2}$ alkyl)amines. Representative pharmaceutically acceptable esters of the invention include $C_{1-7}$ alkyl, $C_{5-7}$ cycloalkyl, phenyl, and phenyl($C_{1-6}$)alkyl esters. Preferred esters include methyl esters.

The invention also includes disclosed compounds having one or more functional groups (e.g., hydroxyl, amino, or carboxyl) masked by a protecting group. See, e.g., Greene and Wuts, Protective Groups in Organic Synthesis, 3rd ed., (1999) John Wiley & Sons, NY. Some of these masked or protected compounds are pharmaceutically acceptable; others will be useful as intermediates. Synthetic intermediates and processes disclosed herein, and minor modifications thereof, are also within the scope of the invention.

HYDROXYL PROTECTING GROUPS

Protection for the hydroxyl group includes methyl ethers, substituted methyl ethers, substituted ethyl ethers, substitute benzyl ethers, and silyl ethers.

Substituted Methyl Ethers

Examples of substituted methyl ethers include methyoxymethyl, methylthiomethyl, *t*-butylthiomethyl, (phenyldimethylsilyl)methoxymethyl, benzyloxymethyl, *p*-methoxybenzyloxymethyl, (4-methoxyphenoxy)methyl, guaiacolmethyl, *t*-butoxymethyl, 4-pentenyloxymethyl, siloxymethyl, 2-methoxyethoxymethyl, 2,2,2-trichloroethoxymethyl, bis (2-chloroethoxy)methyl, 2-(trimethylsilyl)ethoxymethyl, tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl S,S-dioxido, 1-[(2-chloro-4-methyl)phenyl]-4-methoxypiperidin-4-yl, 1,4-dioxan-2-yl, tetrahydrofuranyl, tetrahydrothiofuranyl and 2,3,3a,4,5,6,7,7a-octahydro-7,8,8-trimethyl-4,7-methanobenzofuran-2-yl.

Substituted Ethyl Ethers

Examples of substituted ethyl ethers include 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, and benzyl.

Substituted Benzyl Ethers

Examples of substituted benzyl ethers include *p*-methoxybenzyl, 3,4-dimethoxybenzyl, *o*-nitrobenzyl, *p*-nitrobenzyl, *p*-halobenzyl, 2,6-dichlorobenzyl, *p*-cyanobenzyl, *p*-phenylbenzyl, 2- and 4-picolyl, 3-methyl-2-picolyl N-oxido, diphenylmethyl, *p, p*'-dinitrobenzhydryl, 5-dibenzosuberyl, triphenylmethyl, α-naphthyldiphenylmethyl, *p*-methoxyphenyldiphenylmethyl, di(*p*-methoxyphenyl)phenylmethyl, tri(*p*-methoxyphenyl)methyl, 4-(4'-bromophenacyloxy) phenyldiphenylmethyl, 4,4',4"-tris(4,5-dichlorophthalimidophenyl)methyl, 4,4',4"-tris(levulinoyloxyphenyl)methyl, 4,4', 4"-tris(benzoyloxyphenyl)methyl, 3-(*I*midazol-1-ylmethyl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, and benzisothiazolyl S,S-dioxido.

Silyl Ethers

Examples of silyl ethers include trimethylsilyl, triethylsilyl, triisopropylsilyl, dimethylisopropylsilyl, diethylisopropylsilyl, dimethylthexylsilyl, *t*-butyldimethylsilyl, *t*-butyldiphenylsilyl, tribenzylsilyl, tri-*p*-xylylsilyl, triphenylsilyl, diphenylmethylsilyl, and *t*-butylmethoxyphenylsilyl.

Esters

In addition to ethers, a hydroxyl group may be protected as an ester. Examples of esters include formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, *p*-P-phenylacetate, 3-phenylpropionate, 4-oxopentanoate (levulinate), 4,4-(ethylenedithio)pentanoate, pivaloate, adamantoate, crotonate, 4-methoxycrotonate, benzoate, *p*-phenylbenzoate, 2,4,6-trimethylbenzoate(mesitoate)

Carbonates

Examples of carbonate protecting groups include methyl, 9-fluorenylmethyl, ethyl, 2,2,2-trichloroethyl, 2-(trimethylsilyl)ethyl, 2-(phenylsulfonyl)ethyl, 2-(triphenylphosphonio)ethyl, isobutyl, vinyl, allyl, *p*-nitrophenyl, benzyl, *p*-methoxybenzyl, 3,4-dimethoxybenzyl, *o*-nitrobenzyl, *p*-nitrobenzyl, S-benzyl thiocarbonate, 4-ethoxy-1-naphthyl, and methyl dithiocarbonate.

Assisted Cleavage

Examples of assisted cleavage include 2-iodobenzoate, 4-azidobutyrate, 4-nitro-4-methylpentanoate, *o*-(dibromomethyl)benzoate, 2-formylbenzenesulfonate, 2-(methylthiomethoxy)ethyl carbonate, 4-(methylthiomethoxy)butyrate, and 2-(methylthiomethoxymethyl)benzoate.

Miscellaneous Esters

Examples of miscellaneous esters include 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate(tigloate), *o*-(methoxycarbonyl)benzoate, *p*-P-benzoate, α-naphthoate, nitrate, alkyl N,N,N',N'-tetramethylphosphorodiamidate, N-phenylcarbamate, borate, dimethylphosphinothioyl, and 2,4-dinitrophenylsulfenate

Sulfonates

Examples of sulfonates include sulfate, methanesulfonate(mesylate), benzylsulfonate, and tosylate.

AMINO PROTECTING GROUPS

Protection for the amino group includes carbamates, amides, and special -NH protective groups.
Examples of carbamates include methyl and ethyl carbamates, substituted ethyl carbamates, assisted cleavage carbamates, photolytic cleavage carbamates, urea-type derivatives, and miscellaneous carbamates.

Carbamates

Examples of methyl and ethyl carbamates include methyl and ethyl, 9-fluorenylmethyl, 9-(2-sulfo)fluorenylmethyl, 9-(2,7-dibromo)fluorenylmethyl, 2,7-di-*t*-butyl-[9-(10,10-dioxo-10,10,10,10-tetrahydrothioxanthyl)]methyl, and 4-methoxyphenacyl.

Substituted Ethyl

Examples of substituted ethyl carbamates include 2,2,2-trichloroethyl, 2-trimethylsilylethyl, 2-phenylethyl, 1-(1-adamantyl)-1-methylethyl, 1,1-dimethyl-2-haloethyl, 1,1-dimethyl-2,2-dibromoethyl, 1,1-dimethyl-2,2,2-trichloroethyl, 1-methyl-1-(4-biphenylyl)ethyl, 1-(3,5-di-*t*-butylphenyl)-1-methylethyl, 2-(2'- and 4'-pyridyl)ethyl, 2-(N,N-dicyclohexylcarboxamido)ethyl, *t*-butyl, 1-adamantyl, vinyl, allyl, 1-isopropylallyl, cinnamyl, 4-nitrocinnamyl, 8-quinolyl, N-hydroxypiperidinyl, alkyldithio, benzyl, *p*-methoxybenzyl, *p*-nitrobenzyl, *p*-bromobenzyl, *p*-chlorobenzyl, 2,4-dichlorobenzyl, 4-methylsulfinylbenzyl, 9-anthrylmethyl and diphenylmethyl.

Assisted Cleavage

Examples of assisted cleavage include 2-methylthioethyl, 2-methylsulfonylethyl, 2-(*p*-toluenesulfonyl)ethyl, [2-(1,3-dithianyl)]methyl, 4-methylthiophenyl, 2,4-dimethylthiophenyl, 2-phosphonioethyl, 2-triphenylphosphonioisopropyl, 1,1-dimethyl-2-cyanoethyl, *m*-chloro-*p*-acyloxybenzyl, *p*-(dihydroxyboryl)benzyl, 5-benzisoxazolylmethyl, and 2-(trifluoromethyl)-6-chromonylmethyl.

Photolytic Cleavage

Examples of photolytic cleavage include *m*-nitrophenyl, 3,5-dimethoxybenzyl, *o*-nitrobenzyl, 3,4-dimethoxy-6-nitrobenzyl, and phenyl(*o*-nitrophenyl)methyl.

Urea-Type Derivatives

Examples of urea-type derivatives include phenothiazinyl-(10)-carbonyl derivative, N' -*p*-toluenesulfonylaminocarbonyl, and N'-phenylaminothiocarbonyl.

Miscellaneous Carbamates

Examples of miscellaneous carbamates include *t*-amyl, S-benzyl thiocarbamate, *p*-cyanobenzyl, cyclobutyl, cyclohexyl, cyclopentyl, cyclopropylmethyl, *p*-decyloxybenzyl, diisopropylmethyl, 2,2-dimethoxycarbonylvinyl, *o*-(N,N-dimethylcarboxamido)benzyl, 1,1-dimethyl-3-(N,N-dimethylcarboxamido)propyl, 1,1-dimethylpropynyl, di(2-pyridyl)methyl, 2-furanylmethyl, 2-iodoethyl, isobornyl, isobutyl, isonicotinyl, *p*-(*p*'-methoxyphenylazo)benzyl, 1-methylcyclobutyl, 1-methylcyclohexyl, 1-methyl-1-cyclopropylmethyl, 1-methyl-1-(3,5-dimethoxyphenyl)ethyl, 1-methyl-1-(*p*-phenylazophenyl)ethyl, 1-methyl-1-phenylethyl, 1-methyl-1-(4-pyridyl)ethyl, phenyl, *p*-(phenylazo)benzyl, 2,4,6-tri-*t*-butylphenyl, 4-(trimethylammonium)benzyl, and 2,4,6-trimethylbenzyl.
Examples of amides include:

Amides

N-formyl, N-acetyl, N-chloroacetyl, N-trichloroacetyl, N-trifluoroacetyl, N-phenylacetyl, N-3-phenylpropionyl, N-picolinoyl, N-3-pyridylcarboxamide, N-benzoylphenylalanyl derivative, N-benzoyl, N-*p*-phenylbenzoyl.

Assisted Cleavage

N-*o*-nitrophenylacetyl, N-*o*-nitrophenoxyacetyl, N-acetoacetyl, (N'-dithiobenzyloxycarbonylamino)acetyl, N-3-(*p*-hydroxyphenyl)propionyl, N-3-(*o*-nitrophenyl)propionyl, N-2-methyl-2-(*o*-nitrophenoxy)propionyl, N-2-methyl-2-(*o*-phenylazophenoxy)propionyl, N-4-chlorobutyryl, N-3-methyl-3-nitrobutyryl, N-*o*-nitrocinnamoyl, N-acetylmethionine derivative, N-*o*-nitrobenzoyl, N-*o*-(benzoyloxymethyl)benzoyl, and 4,5-diphenyl-3-oxazolin-2-one.

Cyclic Imide Derivatives

N-phthalimide, N-dithiasuccinoyl, N-2,3-diphenylmaleoyl, N-2,5-dimethylpyrrolyl, N-1,1,4,4-tetramethyldisilylazacyclopentane adduct, 5-substituted 1,3-dimethyl-1,3,5-triazacyclohexan-2-one, 5-substituted 1,3-dibenzyl-1,3,5-triazacyclohexan-2-one, and 1-substituted 3,5-dinitro-4-pyridonyl.

SPECIAL - NH PROTECTIVE GROUPS

Examples of special NH protective groups include

N-Alkyl and N-Aryl Amines

N-methyl, N-allyl, N-[2-(trimethylsilyl)ethoxy]methyl, N-3-acetoxypropyl, N-(1-isopropyl-4-nitro-2-oxo-3-pyrrolin-3-yl), quaternary ammonium salts, N-benzyl, N-di(4-methoxyphenyl)methyl, N-5-dibenzosuberyl, N-triphenylmethyl, N-(4-methoxyphenyl)diphenylmethyl, N-9-phenylfluorenyl, N-2,7-dichloro-9-fluorenylmethylene, N-ferrocenylmethyl, and N-2-picolylamine N'-oxide.

Imine Derivatives

N-1,1-dimethylthiomethylene, N-benzylidene, N-*p*-methoxybenzylidene, N-diphenylmethylene, N-[(2-pyridyl) mesityl]methylene, and N-(N',N'-dimethylaminomethylene).

PROTECTION FOR THE CARBONYL GROUP

Acyclic Acetals and Ketals

Examples of acyclic acetals and ketals include dimethyl, bis(2,2,2-trichloroethyl), dibenzyl, bis(2-nitrobenzyl) and diacetyl.

Cyclic Acetals and Ketals

Examples of cyclic acetals and ketals include 1,3-dioxanes, 5-methylene-1,3-dioxane, 5,5-dibromo-1,3-dioxane, 5-(2-pyridyl)-1,3-dioxane, 1,3-dioxolanes, 4-bromomethyl-1,3-dioxolane, 4-(3-butenyl)-1,3-dioxolane, 4-phenyl-1,3-dioxolane, 4-(2-nitrophenyl)-1,3-dioxolane, 4,5-dimethoxymethyl-1,3-dioxolane, *O*,*O*'-phenylenedioxy and 1,5-dihydro-3H-2,4-benzodioxepin.

Acyclic Dithio Acetals and Ketals

Examples of acyclic dithio acetals and ketals include S,S'-dimethyl, S,S'-diethyl, S,S'-dipropyl, S,S'-dibutyl, S,S'-dipentyl, S,S'-diphenyl, S,S'-dibenzyl and S,S'-diacetyl.

Cyclic Dithio Acetals and Ketals

Examples of cyclic dithio acetals and ketals include 1,3-dithiane, 1,3-dithiolane and 1,5-dihydro-3H-2,4-benzodithiepin.

Acyclic Monothio Acetals and Ketals

Examples of acyclic monothio acetals and ketals include *O*-trimethylsilyl-S-alkyl, *O*-methyl-S-alkyl or -S-phenyl and *O*-methyl-S-2-(methylthio)ethyl.

Cyclic Monothio Acetals and Ketals

Examples of cyclic monothio acetals and ketals include 1,3-oxathiolanes.

MISCELLANEOUS DERIVATIVES

*O*-Substituted Cyanohydrins

Examples of *O*-substituted cyanohydrins include *O*-acetyl, *O*-trimethylsilyl, *O*-1-ethoxyethyl and *O*-tetrahydropyranyl.

Substituted Hydrazones

Examples of substituted hydrazones include N,N-dimethyl and 2,4-dinitrophenyl.

Oxime Derivatives

Examples of oxime derivatives include *O*-methyl, *O*-benzyl and *O*-phenylthiomethyl.

Imines

Substituted Methylene Derivatives, Cyclic Derivatives

Examples of substituted methylene and cyclic derivatives include oxazolidines, 1-methyl-2-(1'-hydroxyalkyl) imidazoles, N,N'-dimethylimidazolidines, 2,3-dihydro-1,3-benzothiazoles, diethylamine adducts, and methylaluminum bis(2,6-di-*t*-butyl-4-methylphenoxide)(MAD)complex.

PROTECTION FOR THE CARBOXYL GROUP

Esters

Substituted Methyl Esters

Examples of substituted methyl esters include 9-fluorenylmethyl, methoxymethyl, methylthiomethyl, tetrahydropyranyl, tetrahydrofuranyl, methoxyethoxymethyl, 2-(trimethylsilyl)ethoxymethyl, benzyloxymethyl, phenacyl, *p*-bromophenacyl, α-methylphenacyl, *p*-methoxyphenacyl, carboxamidomethyl, and N-phthalimidomethyl.

2-Substituted Ethyl Esters

Examples of 2-substituted ethyl esters include 2,2,2-trichloroethyl, 2-haloethyl, ω-chloroalkyl, 2-(trimethylsilyl) ethyl, 2-methylthioethyl, 1,3-dithianyl-2-methyl, 2-(p-nitrophenylsulfenyl)ethyl, 2-(p-toluenesulfonyl)ethyl, 2-(2'-pyridyl) ethyl, 2-(diphenylphosphino)ethyl, 1-methyl-1-phenylethyl, *t*-butyl, cyclopentyl, cyclohexyl, allyl, 3-buten-1-yl, 4-(trimethylsilyl)-2-buten-1-yl, cinnamyl, α-methylcinnamyl, phenyl, *p*-(methylmercapto)phenyl and benzyl.

Substituted Benzyl Esters

Examples of substituted benzyl esters include triphenylmethyl, diphenylmethyl, bis(o-nitrophenyl)methyl, 9-anthrylmethyl, 2-(9,10-dioxo)anthrylmethyl, 5-dibenzosuberyl, 1-pyrenylmethyl, 2-(trifluoromethyl)-6-chromylmethyl, 2,4,6-trimethylbenzyl, *p*-bromobenzyl, *o*-nitrobenzyl, *p*-nitrobenzyl, *p*-methoxybenzyl, 2,6-dimethoxybenzyl, 4-(methylsulfinyl)benzyl, 4-sulfobenzyl, piperonyl, 4-picolyl and *p*-P-benzyl.

Silyl Esters

Examples of silyl esters include trimethylsilyl, triethylsilyl, *t*-butyldimethylsilyl, *i*-propyldimethylsilyl, phenyldimethylsilyl and di-*t*-butylmethylsilyl.

Activated Esters

Examples of activated esters include thiols.

Miscellaneous Derivatives

Examples of miscellaneous derivatives include oxazoles, 2-alkyl-1,3-oxazolines, 4-alkyl-5-oxo-1,3-oxazolidines, 5-alkyl-4-oxo-1,3-dioxolanes, ortho esters, phenyl group and pentaaminocobalt(III) complex.

Stannyl Esters

Examples of stannyl esters include triethylstannyl and tri-*n*-butylstannyl.

AMIDES AND HYDRAZIDES

Amides

Examples of amides include N,N-dimethyl, pyrrolidinyl, piperidinyl, 5,6-dihydrophenanthridinyl, *o*-nitroanilides, N-7-nitroindolyl, N-8-Nitro-1,2,3,4-tetrahydroquinolyl, and *p*-P-benzenesulfonamides.

Hydrazides

Examples of hydrazides include N-phenyl and N,N'-diisopropyl hydrazides.

E. Chemical Examples

Example 1

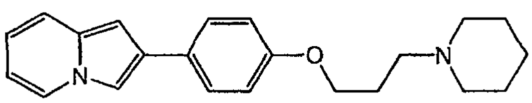

2-(4-Piperidinopropoxyphenyl)indolizine
$K_i$ = 4 nM

Step A Preparation of 2-(4-methoxyphenyl)indolizine

A solution of 2-bromo-4'-methoxyacetophenone (10 mmol, 2.29 g) and 2-picoline (10 mmol, 0.986 mL) in acetone (50 mL) was heated at reflux for 4 hours. The quaternary salt was precipitated out. The salt was collected, redissolved in hot (60 - 90° C) water (50 mL), and treated with potassium carbonate (10 mmol, 1.38g). The mixture was heated at 80° C for 8 hours. After filtration and drying in vacuo, the title compound (2.2 g) was collected.

Step B Preparation of 2-(4-hydroxyphenyl)indolizine

A mixture of 2-(4-methoxyphenyl)indolizine (4 mmol, 0.892 g) and sodium ethanethiolate (8 mmol, 0.673 g) in N, N-dimethylformamide (10 mL) was heated at 80 °C for 8 h. The solvent was evaporated and the residue dried in vacuo. water (100 mL) was added, and a pale white solid formed. After filtration and drying in vacuo, the title compound (2 g) was collected.

Step C Preparation of 2-(4-piperidinopropoxyphenyl)indolizine

The mixture of 2-(4-hydroxyphenyl)indolizine (0.1 mmol, 21 mg), 1-piperidinepropanyl chloride, and sodium methoxide (0.3 mmol, 21 mg) in N, N-dimethylformamide (2 mL) was heated at 80 °C for 8 h. The solvent was evaporated and water (20 mL) was added. After extraction with dichloromethane (3 x 15 mL), drying over sodium sulfate, evaporation, the title compound (31 mg) was obtained. $^1$H NMR ($CDCl_3$, 400MHz) δ 7.81 (dd, 1 H, *J* = 1.1, 7.0 Hz), 7.38 (td, 2H, *J* = 6.7, 2.1 Hz), 7.30 (broad, 1H), 7.25 (broad d, 1 H, *J* = 9.0 Hz), 6.86 (td, 2H, *J* = 6.7, 2.1 Hz), 6.65 (m, 2H), 6.46 (tt, 1 H, *J* = 6.7, 1.1 Hz), 3.95 (t, 2H, *J* = 6.4 Hz), 2.40 (m, 6H),1.95 (m, 2H), 1.65 ( broad m, 4H), 1.40 (broad, 2H); EIMS *m*/*z* 335 (M + $H^+$).

Example 2

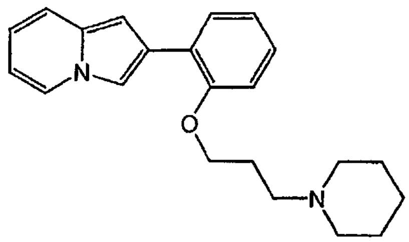

2-(2-Piperidinopropoxyphenyl)indolizine
$K_i$ = 855 nM

Step A Preparation of 2-(2-methoxyphenyl)indolizine

A solution of 2-bromo-2'-methoxyacetophenone (10 mmol, 2.29 g) and 2-picoline (10 mmol, 0.986 mL) in acetone (50 mL) was heated at reflux for 8 hours. The quaternary salt precipitated out. The salt was collected and redissolved in hot (60 - 90° C) water (50 mL) and treated with potassium carbonate (10 mmol, 1.38g). The mixture was heated at 80 °C for 3 days. After filtration and drying in vacuo, the title compound (1.4 g) was collected.

Step B Preparation of 2-(2-hydroxyphenyl)indolizine

A mixture of 2-(2-methoxyphenyl)indolizine (2.2 mmol, 0.50 g) and sodium ethanethiolate (4.4 mmol, 0.46 g) in N, N-dimethylformamide (15 mL) was heated at 130 °C for 8 hours. The solvent was evaporated and the residue was dried in vacuo. Water (100 mL) was added, and pale white solid formed. After filtration and drying in vacuo, the title compound (0.10 g, 21.3%) was collected.

Step C Preparation of 2-(2-piperidinopropoxyphenyl)indolizine

The mixture of 2-(2-hydroxyphenyl)indolizine (0.4 mmol, 84.4 mg), 1-piperidinepropanyl chloride, and sodium methoxide (1.2 mmol, 65 mg) in N, N-dimethylformamide (6 mL) was heated at 80 °C for 8 hours. The solvent was evaporated and water (30 mL) was added. After extraction with dichloromethane (3 x 20 mL), drying over sodium sulfate, evaporation, and purification by preparative TLC on silica gel (10% methanol/dichloromethane), ,the title compound (54 mg, 40.4%) was obtained. $^1$H NMR (400 MHz, $CDCl_3$) δ 7.88 (br d, J =7.0, 1 H), 7.85 (d, J = 1.0, 1H), 7.78 (dd, J = 7.6, 1.7, 1 H), 7.33 (br d, J = 9.0, 1 H), 7.21 (ddd, J = 8.2, 7.5, 1.7, 1 H), 7.03-6.97 (m, 2H), 6.79 (br s, 1H), 6.61 (ddd, J = 9.0, 6.5, 1.0, 1 H), 6.42 (dt, J = 6.9, 1.2, 1 H), 4.12 (t, J = 6.4, 2H), 2.56 (m, 2H), 2.43 (br s, 3H), 2.31-2.10 (m, 2H), 1.63-1.61 (m, 5H). $^{13}$C NMR (400 MHz, $CDCl_3$) δ 155.9, 132.3, 128.9, 127.3, 125.1, 124.9, 124.0, 120.8, 118.8, 116.8, 112.5, 112.3, 110.1, 98.2, 66.9, 56.3, 54.7, 26.9, 25.9, 24.3.

Example 3

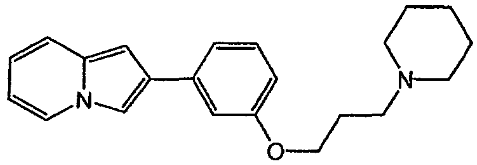

2-(3-Piperidinopropoxyphenyl)indolizine
$K_i$ = 19 nM

Step A Preparation of 2-(3-methoxyphenyl)indolizine

A solution of 2-bromo-3'-methoxyacetophenone (10 mmol, 2.29 g) and 2-picoline (10 mmol, 0.986 mL) in acetone (50 mL) was heated at reflux for 8 hours. The quaternary salt precipitated out. The salt was collected and redissolved in hot (60 - 90° C) water (50 mL) and potassium carbonate (10 mmol, 1.38g) was added. The mixture was heated at 80° C for 3 days. After filtration and drying in vacuo, the title compound (1.35 g) was collected.

Step B Preparation of 2-(3-hydroxyphenyl)indolizine

A mixture of 2-(3-methoxyphenyl)indolizine (3.65 mmol, 0.815 g) and sodium ethanethiolate (7.3 mmol, 0.768 g) in N, N-dimethylformamide (22 mL) was heated at 80° C for 8 hours. The solvent was evaporated and the residue dried in vacuo. Water (200 mL) was added and a pale white solid was formed. After filtration and drying in vacuo, the title compound (0.44 g) was collected.

Step C Preparation of 2-(3-piperidinopropoxyphenyl)indolizine

A mixture of 2-(3-hydroxyphenyl)indolizine (0.48 mmol, 100 mg), 1-piperidinepropanyl chloride, and sodium methoxide (1.43 mmol, 77 mg) in N, N-dimethylformamide (5 mL) was heated at 80° C for 8 hours. The solvent was evaporated and water (25 mL) was added. After extraction with dichloromethane (3 x 15 mL), drying over sodium sulfate, evaporation, and recrystallization (ethyl acetate/hexane), the title compound (69 mg) was obtained. $^1$H NMR (400 MHz, $CDCl_3$) δ 7.88 (dd, *J* = 7.0, 1.0, 1H), 7.56 (d, *J* = 1.3, 1 H), 7.33 (br d, *J* = 9.0, 1 H), 7.31-7.22 (m, 2H), 7.20-7.18 (m, 1H), 6.80(ddd, *J* = 8.0, 2.5, 1.1, 1H), 6.67 (br s, 1H), 6.64 (ddd, *J* = 9.0, 6.5, 1.0, 1 H), 6.44 (dt, *J* = 6.8, 1.2, 1 H), 4.06 (t, *J* = 6.4, 2H), 2.53-2.50 (m, 2H), 2.43 (br s, 4H), 2.02 (m, 2H), 1.60 (quint, *J* = 5.5, 4H), 1.45 (m,2H). 13C NMR (400 MHz, CDCl3) δ 159.4, 136.7, 133.5, 129.6, 129.3, 125.0, 119.0, 118.7, 117.3, 112.54, 112.50, 110.5, 109.3, 96.7, 66.4, 56.0, 54.6 ,26.8, 25.8, 24.4.

Example 4

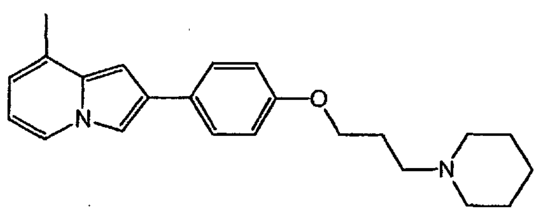

$K_i$ = 73 nM
8-Methyl-2-[4-(3-piperidinylpropoxy)-phenyl]-indolizine

Step A Preparation of 8-methyl-2-(4-Methoxyphenyl)-indolizine

A solution of 2-bromo-4'-methoxyacetophenone (22 mmol, 5 g) and 2,3-lutidine (22 mmol, 2.5 mL) in acetone (50 mL) was heated at reflux temperature for 8 hours. The quaternary salt precipitated out and was washed with cold

acetone. The salt was collected and redissolved in hot water (50 mL). Potassium carbonate (55 mmol, 7.6g) was added and the mixture was heated at 100 °C for 8 hours. The filtrated solid was collected and dried in vacuo to afford the title compound (5.02 g, 96 %) as white solid, which was used without purification.

Step B Preparation of 8-methyl-2-(4-Hydroxyphenyl)indolizine

To a solution of 8-Methyl-2-(4-Methoxyphenyl)-indolizine (9.1 mmol, 2.16 g) in acetic acid (13 mL) was added 48% HBr (32 mL) slowly. The mixture was stirred and heated at 100 °C for 8 hours. Solvent was removed in vacuo to give the title compound, which was sued without purification.

Step C Preparation of 8-methyl-2-[4-(3-piperidinylpropoxy)-phenyl]-indolizine

The mixture of 5-methyl-2-(4-hydroxyphenyl)indolizine (9.1 mmol, 2.0 g), 1-piperidinepropanyl chloride (9.1 mmol, 1.8 g), and sodium methoxide (45.5 mmol, 2.5 g) in N, N-dimethylformamide (40 mL) was heated at 60 °C for 8 hours. Next day, 1-piperidinepropanyl chloride (0.8 g) and sodium methoxide (1 g) was added and the reaction mixture was stirred and heated at 50 °C for 8 hours. N,N-dimethylformamide was evaporated. Then water (200 mL) was added. After extraction with ethyl acetate (3 x 150 mL), dried over sodium sulfate, and evaporated. Recrystallization using ethyl acetate and hexane. The title compound (11.8 mg, 0.4%) was obtained. $^1$H NMR (400 MHz, $CDCl_3$) δ 8.15 (d, *J* = 6.5, 1 H), 7.93 (d, *J* = 1.6, 1H), 7.71 (d, *J* = 8.7, 2H), 7.00 (d, *J* = 8.7, 2H), 6.76 (br s, 1 H), 6.49 (m, 2H), 4.07 (t, *J* = 6.4, 2H), 3.49 (s, 3H), 2.45-2.36 (m, 6H), 1.91 (quint, *J* = 6.7, 2H), 1.55-1.50 (m, 4H), 1.41-1.35 (m, 2H).

Example 5

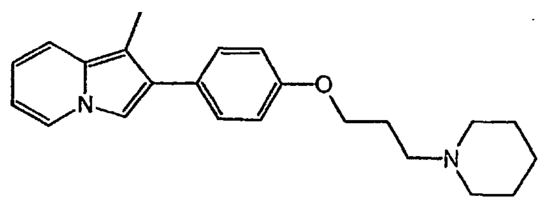

1-Methyl-2-[4-(3-piperidinylpropoxy)-phenyl]-indolizine
$K_i$ = 9 nM

Step A Preparation of 1-methyl-2-(4-methoxyphenyl)indolizine

A solution of 2-bromo-4'-methoxyacetophenone (19 mmol, 4.4 g) and 2-ethylpyridine (19 mmol, 2.06 mL) in acetone (50 mL) was heated at reflux temperature for 8 hours. The quaternary salt precipitated out and was washed with cold acetone. The salt was collected and redissolved in hot water (50 mL). Potassium carbonate (55 mmol, 7.6g) was added and the mixture was heated at 100 °C for 8 hours. The filtrated solid was collected and dried in vacuo to afford the title compound (3.7 g, 82%) as brown solid, which was used without purification.

Step B Preparation of 1-methyl-2-(4-hydroxyphenyl)indolizine

To a solution of 1-methyl-2-(4-methoxyphenyl)indolizine (15.6 mmol, 3.7 g) in acetic acid (15 mL) was added 48% HBr (55 mL) slowly. The mixture was stirred and heated at 100 °C for 8 hours. Solvent was removed in vacuo to give the title compound, which was used without purification.

Step C Preparation of 1-methyl-2-[4-(3-piperidinopropoxy)-phenyl]indolizine

To a solution of 1-methyl-2-(4-hydroxyphenyl)indolizine (0.46 mmol, 0.102 g) in 4 mL of N,N-dimethylformamide, was added sodium methoxide (1,84, 0.099 g). The mixture was stirred and heated at 40 °C for 2 hours. 1-piperidinepropanyl chloride (0.46 mmol, 0.091 g) was added and the reaction mixture was heated at 60 °C for 8 hours. N, N-dimethylformamide was evaporated. Then water (40 mL) was added. After extraction with ethyl acetate (3 x 30 mL), dried over magnesium sulfate, evaporated. Purification via preparative thin layer chromatography on alumina using 20% ethyl acetate in hexane as eluant gave the title compound (48.6 mg, 30%). $^1$H NMR (400 MHz, $CDCl_3$) δ 7.82 (d,

J = 6.9, 1 H), 7.42 (d, J = 8.7, 2H), 7.32-7.29 (m, 2H), 6.96 (d, J = 8.7, 2H), 6.58 (dd, J = 9.0, 6.4, 1H), 6.38 (dt, J = 6.5, 1.0, 1 H), 4.04 (t, J = 6.4, 2H), 2.52-2.49 (m, 2H), 2.43-2.39 (m, 6H), 2.04-1.98 (m, 2H), 1.63-1.57(m, 5H), 1.45-1.44 (m, 2H).

Example 6

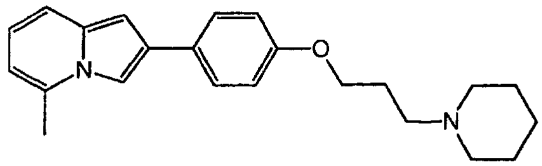

5-Methyl-2-(4-piperidinylpropoxyphenyl)indolizine
$K_i$ = 65 nM

Step A Preparation of 5-methyl-2-(4-methoxyphenyl)indolizine

A solution of 2-bromo-4'-methoxyacetophenone (25 mmol, 6.0 g) and 2,6-lutidine (25 mmol, 3.0 mL) in acetone (60 mL) was heated at reflux temperature for 8 hours. The quaternary salt precipitated out and was washed with cold acetone. The salt was collected and redissolved in hot water (60 mL). Potassium carbonate (62.5 mmol, 8.6 g) was added and the mixture was heated at 100 °C for 8 hours. The filtrated solid was collected and dried in vacuo to afford the title compound (3.05 g, 52%) as greyish solid, which was used without purification.

Step B Preparation of 5-methyl-2-(4-hydroxyphenyl)indolizine

To a solution of 5-methyl-2-(4-methoxyphenyl)indolizine (12.8 mmol, 3.05 g) in acetic acid (15 mL) was added 48% HBr (45 mL) slowly. The mixture was stirred and heated at 100 °C for 8 hours. Solvent was removed in vacuo to give the title compound, which was used without purification.

Step C Preparation of 5-methyl-2-(4-piperidinylpropoxyphenyl)indolizine

To a solution of 5-methyl-2-(4-hydroxyphenyl)indolizine (0.61 mmol, 0.136 g) in 5 mL of N,N-dimethylformamide, was added sodium methoxide (1,84 mmol, 0.099 g). The mixture was stirred and heated at 80 °C for 2 hours. 1-piperidinepropanyl chloride (0.61 mmol, 0.121 g) was added and the reaction mixture was heated at 60 °C for 3 days. N,N-dimethylformamide was evaporated. Then water (40 mL) was added. After extraction with ethyl acetate (3 x 30 mL), dried over magnesium sulfate, evaporated. Purification via preparative thin layer chromatography on alumina using 20% ethyl acetate in hexane as eluant gave the title compound (14.6 mg, 6.9%). $^1H$ NMR (400 MHz, $CDCl_3$) δ 7.61 (d, *J* = 8.6, 2H), 7.39 (s, 1 H), 7.30 (d, *J* = 8.8, 1H), 6.94 (d, *J* = 8.6, 2H), 6.72-6.65 (m, 2H), 6.34 (d, *J* = 6.5, 1 H), 4.04 (t, *J* = 6.3, 2H), 2.53-2.36 (m, 9H), 2.04-1.97 (m, 2H), 1.62-1.58 (m, 4H), 1.46 (br s, 2H).

Example 7

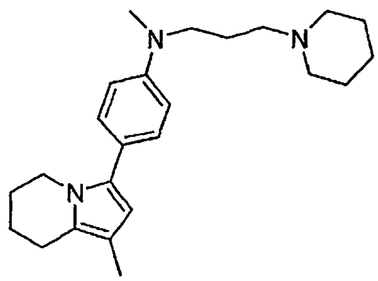

Methyl-[4-(1-methyl-5,6,7,8-tetrahydro-indolizin-3-yl)-phenyl]-(3-piperidin-1-yl-propyl)-amine
$K_i$ = 22 nM

Step A: Preparation of 2-[2-(4-nitro-phenyl)-2-oxo-ethyl]-cyclohexane-1,3-dione

A mixture of 1,3-cyclohexandione (1.38 g), 2-bromo-4'-nitroacetophenone (3.0 g), and potassium carbonate (2.21 g) in chloroform (25 mL) was stirred at ambient temperature for 2 days. The mixture was filtered and the resulting solid dissolved in water and neutralized with concentrated HCl to afford the title compound (1.29 g).

Step B: Preparation of 2-methyl-2-[2-(4-nitro-phenyl)-2-oxo-ethyl]-cyclohexane-1,3-dione

The product of Step A was dissolved in dimethylformamide (26 mL) and treated sequentially with DBU (1.94 mL) and iodomethane (1.21 mL). The mixture was stirred at ambient temperature for 24 h and then diluted with water (40 mL). The mixture was extracted with dichloromethane and the combined organic extracts were washed with brine, dried over sodium sulfate, filtered and concentrated. The residue was purified via silica gel chromatography (EtOAc/ hexanes) to give the title compound (1.133 g).

Step C Preparation of 1-methyl-3-(4-nitro-phenyl)-7,8-dihydro-6H-indolizin-5-one

A solution of the product of Step B (1.133 g) in acetic acid (10 mL) was treated with ammonium acetate (4.5 g) and the mixture stirred at 120° C for 4 hours. The mixture was concentrated *in vacuo* and diluted with water. The aqueous mixture was extracted with dichloromethane and the combined organic layers were washed with saturated aqueous sodium bicarbonate solution, dried over sodium sulfate, filtered and concentrated. The residue was purified via silica gel chromatography (EtOAc/hexanes) to afford the title compound (0.1603 g). Alternatively, the product of step (B) can be dissolved in an excess of liquid ammonia at -78° and stirred for 2 hours. After isolating the product by evaporation, the residue is heated to 100° C and then cooled to afford the title compound.

Step D: Preparation of 3-(4-amino-phenyl)-1-methyl-7,8-dihydro-6H-indolizin-5-one

A solution of the product of Step C (0.160 g) in ethanol (10 mL) was treated with tin (II) chloride (0.4 g) and the mixture stirred at 78° C for 12 hours. The mixture was concentrated *in vacuo* and the residue partitioned between dichlormethane and saturated aqueous sodium bicarbonate solution. The organic layer was separated, dried over sodium sulfate, filtered and concentrated to an orange foam that was used without further purification.

Step E: Preparation of N-[4-(1-methyl-5-oxo-5,6,7,8-tetrahydro-indolizin-3-yl)-phenyl]-3-piperidin-1-yl-propionamide

A solution of the product of Step D (0.078 g) in dichloromethane (2 mL) was treated with EDCI (0.0685 g), HOBt (0.0486 g), DMAP (0.044 g), 3-piperidinepropionic acid (0.0566 g) and stirred at ambient temperature for 2 days. The mixture was diluted with dichloromethane and washed with saturated aqueous sodium bicarbonate solution. The combined organic layers were dried over sodium sulfate, filtered and concentrated. The residue was purified via silica gel chromatography (EtOAc/hexanes) to yield the title compound (0.06 g).

Step F: Preparation of N-methyl-N-[4-(1-methyl-5-oxo-5,6,7,8-tetrahydro-indolizin-3-yl)-phenyl]-acrylamide

The product of Step E (0.049 g) was dissolved in dimethylformamide (2 mL) was treated with 1.0M lithium hexamethyldisilazane in hexanes (0.194 mL) at 0° C. The mixture was stirred for 20 minutes at 0° C and then treated with iodomethane (0.012 mL). The mixture was allowed to warm to ambient temperature and then diluted with water. The mixture was extracted with dichloromethane and the combined organic extracts washed with brine, dried over sodium sulfate, filtered and concentrated. The residue was purified via silica gel chromatography (EtOAc/hexanes) to give the title compound (0.025 g).

Step G: Preparation of N-methyl-N-[4-(1-methyl-5-oxo-5,6,7,8-tetrahydro-indolizin-3-yl)-phenyl]-3-piperidin-1-yl-propionamide

A solution of the product of Step F dissolved in toluene (1 mL) was treated with piperidine (0.009 mL) and stirred at 80° C for 16 hours. The mixture was concentrated in vacuo and purified via silica gel chromatography (EtOAc/MeOH) to afford the title compound (0.029 g).

Step H: Preparation of methyl-[4-(1-methyl-5,6,7,8-tetrahydro-indolizin-3-yl)-phenyl]-(3-piperidin-1-yl-propyl)-amine

A solution of the product of Step G (0.018 g) dissolved in THF (1 mL) was treated with 1.0M borane in THF (1 mL) and stirred at 60°C for 12 hours. The mixture was concentrated in vacuo and dissolved in 1 N HCl (6 mL) and stirred at 100°C for 1 hour. The mixture was neutralized with 25% NaOH and extracted with dichloromethane. The combined organic extracts were dried over sodium sulfate, filtered and concentrated. The residue was purified via silica gel chromatography (EtOAc/MeOH) to afford the title compound (0.0067 g). $^1H$ NMR ($CD_3OD$) δ 7.16 (m, 2 H), 6.75 (m, 2 H), 5.79 (s, 1 H), 3.81 (m, 2 H), 3.37 (m, 2 H), 2.93 (m, 2 H), 2.68 (m, 2 H), 2.42 (m, 6 H), 1.89 (s, 3 H), 1.82 (m, 6 H), 1.61 (m, 5 H), 1.48 (m, 2H).

Example 8

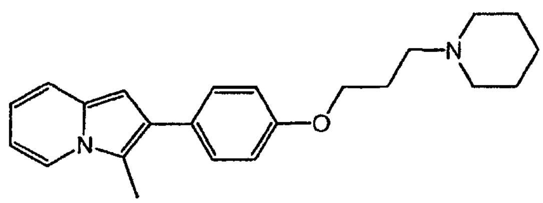

3-Methyl-2-(4-piperidinylpropoxyphenyl)indolizine
$K_i$ = 1 nM

Step A Preparation of 4'-(3-chloropropoxy)propiophenone

A mixture of 4-hydroxypropiophenone (66.6 mmol, 10.0 g) and 1-bromo-3-chloropropane (79.9 mmol, 7.9 mL) in acetone (350 mL) was treated with potassium carbonate (106.6 mmol, 14.7 g). The mixture was stirred at reflux temperature for 16 hours. The reaction was cooled to ambient temperature and filtered. The filtrate was concentrated in vacuo. The residue was dissolved in diethyether, washed with water, dried over sodium sulfate, filtered and concentrated in vacuo to yield the title compound (15.0 g, 100%).

Step B Preparation of 2-bromo-4'-(3-chloropropoxy)propiophenone

Bromine (44 mmol, 7.05 g) was added dropwise to 50 mL of dioxane. The resulting solution was added dropwise to a solution of 4'-(3-chloropropoxy)propiophenone in dioxane (40 mL). The mixture was stirred at rt for 8 hours. The reaction mixture was diluted with water (300 mL) and extracted with chloroform (500 mL), washed with brine, dried over sodium sulfate and concentrated in vacuo yield the title compound (14.0 g, 100%).

Step C Preparation of 3-methyl-2-(4'-(3-chloropropoxy))indolizine

A solution of 2-bromo-4'-(3-chloropropoxy)propiophenone (44 mmol, 13.4 g) and 2-picoline (52.8 mmol, 5.21 mL) in acetone (300 mL) was heated at reflux for 2 days. The precipitated glue was washed with cold acetone, collected and redissolved in hot water (300 mL). Insoluble solids were filtered out. Potassium carbonate (132 mmol, 18.24 g) was added and the mixture was heated at 100 °C for 4 hours. The mixture was extracted with chloroform (500 mL), dried over sodium sulfate, filtered, and concentrated. Purification via silica gel chromatography in 5% ethyl acetate/hexane to result the title compound (2.04 g, 15.6%).

Step D Preparation of 3-methyl-2-(4-piperidinopropoxyphenyl)indolizine

A solution of 3-methyl-2-(4'-(3-chloropropoxy))indolizine (6.8 mmol, 2.04 g) in 20 mL of piperidine was heated at 100°C for 1.5 hours. The resulting mixture was then stirred at rt for 2 days. The reaction mixture was concentrated in vacuo. The residue was dissolved in ethyl acetate (20 mL) and extracted with sodium bicarbonate(2 x 20 mL), dried over sodium sulfate, filtered and concentrated to result the title compound (2.37 g, 100%). $^1$H NMR (400 MHz, $CDCl_3$) δ 7.70 (d, *J* = 7.1, 1 H), 7.42 (d, *J* = 8.6, 2H), 7.38 (d, *J* = 8.9, 1H), 7.00 (d, *J* = 8.6, 2H), 6.68-6.64 (m, 1H), 6.57-6.53 (m, 2H), 4.06 (t, *J* = 6.4, 2H) 2.53-2.22 (m, 9H), 2.06-1.98 (m, 2H), 1.61 (quint, *J* = 5.6, 4H), 1.46 (m, 2H). $^{13}$C NMR (400 MHz, $CDCl_3$) δ 158.1, 132.1, 130.4, 129.5, 127.8, 122.1, 119.1, 116.4, 116.2, 115.0, 110.5, 98.6, 70.0, 56.5, 55.1, 27.4, 26.4, 24.9, 10.7

Example 8 was initially prepared using the method described in Example 4, steps A - C. However, during step B of Example 8, a by-product was formed, which was carried on with the desired material to step C. The final by-product (A) was inseparable from the actual product (Example 8). Using NMR, mass spect and UV, the following structure was proposed to be the by-product.

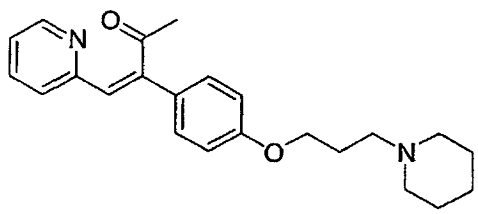

(A)

3-[4-(3-Piperidin-1-yl-propoxy)-phenyl]-4-pyridin-2-yl-but-3-en-2-one

An alternative synthetic method was then devised to synthesize Example 8 <u>without</u> forming compound(A), and this is the method that is described as Example 8, Steps A - C.

Example 9

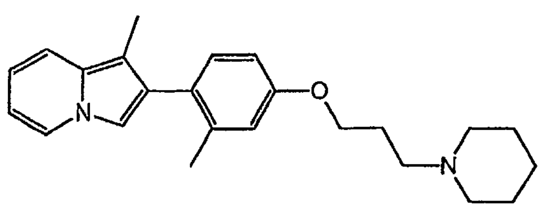

1-Methyl-2-(2-methyl-4-(3-piperidinylpropoxyphenyl))indolizine
$K_i$ = 7 nM

Step A Preparation of 4'-methoxy-2'-methylacetophenone

To a solution of 4'-hydroxy-2'-methylacetophenone (26.7 mmol, 4.00 g) in 20 mL of acetone, iodomethane (59 mmol, 3.7 mL) and potassium carbonate (59 mmol, 8.2 g) were added. The reaction mixture was stirred and heated

at 50 °C for 8 hours. Acetone was removed in vacuo, then water was added, and extracted with dichloromethane. Organic layer was dried over sodium sulfate, filtered and concentrated to give the title compound (4.4g, 100%).

Step B Preparation of 2-bromo-4'-methoxy-2'-methylacetophenone

To a solution of 4'-methoxy-2'-methylacetophone (33.3 mmol, 5.47 g) in ether (50 mL), bromide (33.3 mmol, 1.72 g) was added dropwise. Reaction mixture was stirred at rt for 8 hours. Then poured into sodium bicarbonate solution (100 mL). Then washed with water and extracted with dichloromethane (3 x 100 mL). The organic layer was dried over sodium sulfate, filtered and concentrated. The residue was purified via silica gel chromatography in 15 % ethyl acetate/hexane to result the title compound with a 3:1 mixture of product and starting material.

Step C Preparation of 3-methyl-2-(2-methyl-4-methoxyphenyl)indolizine

A solution of 2-bromo-4'-methoxy-2'-methylacetophenone (13.6 mmol, 3.3 g) and ethylpiperidine (13.6 mmol, 1.5 ml) in acetone (50 mL) was heated at reflux for 8 hours. The quaternary salt was precipitated out, and was washed with cold acetone. The salt was collected and redissolved in hot water (50 mL). Potassium carbonate (40.8 mmol, 5.6 g) was added and the mixture was heated at 100 °C for 8 hours. The filtrated solid was collected and dried in vacuo to afford the title compound (2.08 g, 61 %), which was used without purification.

Step D Preparation of 3-methyl-2-(2-methyl-4-hydroxyphenyl)indolizine

To a solution of 3-methyl-2-(2-methyl-4-methoxyphenyl)indolizine in acetic acid (8.3 mL) was added 48% HBr (30 mL) slowly. The mixture was stirred and heated at 100 °C for 8 hours. Solvent was removed in vacuo to afford the title compound, which was used without purification.

Step E Preparation of 3-methyl-2-(2-methyl-4-piperidino-propoxyphenyl)-indolizine

To a solution of 3-methyl-2-(2-methyl-4-hydroxyphenyl)indolizine (0.45 mmol, 0.107 g) in 10 mL of N,N-dimethylformamide, was added sodium *tert*-butoxide (1.8 mmol , 0.173 g). The mixture was stirred and heated at 40 °C for 2 hours. 1-piperidinepropanyl chloride (0.45 mmol, 0.090 g) was added and the reaction mixture was heated at 60 °C for 8 hours. N,N-dimethylformamide was evaporated. Then water (40 mL) was added. After extraction with ethyl acetate (3 x 30 mL), dried over magnesium sulfate, evaporated to afford the title compound (0.081, 50%). $^1$H NMR (400 MHz, $CDCl_3$) $\delta$ 7.76 (dt, J = 6.9, 1.0, 1 H), 7.72 (d, J = 9.0, 1 H), 7.10 (s, 1 H), 7.06 (d, J = 8.3, 1 H), 6.77 (d, J = 2.6, 1 H), 6.69 (dd, J = 8.3, 2.6, 1 H), 6.52 (dd, J = 9.0, 6.4, 1 H), 6.35-6.31 (m, 1H), 3.97 (t, J = 6.4, 2H), 2.47-2.43 (m, 2H), 2.36 (br s, 4H), 2.12 (s, 3H), 2.09 (s, 3H), 1.96-1.91 (m, 2H), 1.56-1.52 (m, 2H), 1.38 (br s, 2H).

Example 10

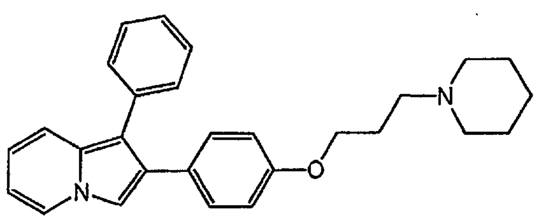

1-Phenyl-2-[4-(3-piperidinylpropoxy)phenyl]indolizine
$K_i$ = 7 nM

Step A Preparation of 1-phenyl-2-(4-methoxyphenyl)indolizine

A solution of 2-bromo-4'-methoxyacetophenone (19.8 mmol, 4.53 g) and 2-benzylpyridine (19.8 mmol, 3.35 g) in acetone (50 mL) was heated at reflux temperature for 8 hours. The quaternary salt precipitated out and was washed with cold acetone. The salt was collected and redissolved in hot water (50 mL). Potassium carbonate (59.4 mmol, 8.2 g) was added and the mixture was heated at 100 °C for 8 hours. After cooling to rt and extracted with

dichloromethane, dried over sodium sulfate, filtered and concentrated to gave the title compound (4.04 g, 69%) as green form, which was used without purification.

Step B Preparation of 1-phenyl-2-(4-hydroxyphenyl)indolizine

To a solution of 1-phenyl-2-(4-methoxyphenyl)indolizine (13.5 mmol, 4.04 g) in acetic acid (13.5 mL) was added 48% HBr (47.5 mL) slowly. The mixture was stirred and heated at 100 °C for 8 hours. Solvent was removed in vacuo to give the title compound, which was used without purification.

Step C Preparation of 1-phenyl-2-[4-(3-piperidinylpropoxy)phenyl]indolizine

To a solution of 1-phenyl-2-(4-hydroxyphenyl)indolizine (0.41 mmol, 0.082 g) in 10 mL of N,N-dimethylformamide, was added sodium *tert*-butoxide (1.65 mmol, 0.159 g). The mixture was stirred and heated at 40 °C for 2 hours. 1-piperidinepropanyl chloride (0.41 mmol, 0.82 g) was added and the reaction mixture was heated at 60 °C for 16h. N,N-dimethylformamide was evaporated.

Then water (40 mL) was added. After extraction with ethyl acetate (3 x 30 mL), dried over magnesium sulfate, evaporated to give the title compound (0.073g, 47%). $^1H$ NMR (400 MHz, $CDCl_3$) δ 7.83 (dt, *J* = 7.0, 1.0, 1H), 7.40 (d, *J* = 9.1, 1H), 7.33 (s, 1 H), 7.27-7.26 (m, 4H), 7.25-7.13 (m, 3H), 7.14 (d, *J* = 8.8, 2.9, 2H), 6.59 (ddd, *J* = 9.1, 6.5, 1.0, 1H), 6.41 (dt, *J* = 6.7, 1.1, 1H), 3.93 (t, *J* = 6.3, 2H), 2.45-2.36 (m, 6H), 1.96-1.89 (m, 2H), 1.58-1.52 (m, 4H), 1.40-1.18 (m, 2H). $^{13}C$ NMR (400 MHz, $CDCl_3$) δ 157.9, 135.5, 131.2, 130.4, 130.3, 128.5, 128.2, 127.8, 125.8, 125.1, 118.1, 118.0, 114.5, 112.4, 111.1, 111.0, 66.5, 56.2, 54.8, 26.9, 25.9, 24.5.

<u>Example 11</u>

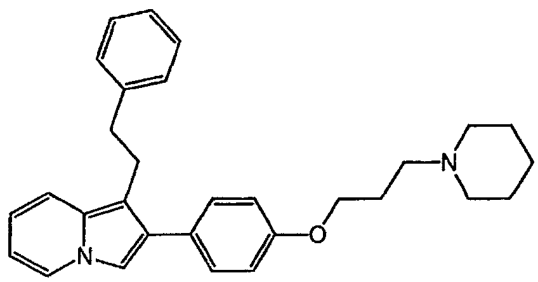

1-Phenethyl-2-[4-(3-piperidinylpropoxy)phenyl]indolizine
Ki = 5 nM

Step A Preparation of 1-phenethyl-2-(4-methoxyphenyl)indolizine

A solution of 2-bromo-4'-methoxyacetophenone (14.1 mmol, 3.24 g) and 3-phenylpropyl pyridine (14.1 mmol, 2.79 g) in acetone (50 mL) was heated at reflux temperature for 8 hours. The quaternary salt precipitated out, and was washed with cold acetone. The salt was collected and redissolved in hot water (50 mL). Potassium carbonate (59.4 mmol, 8.2 g) was added and the mixture was heated at 100 °C for 8 hours. After cooling to rt, solid was filtered and dried in vacuo to afford the title compound (3.80 g, 83%) as black solid, which was used without purification.

Step B Preparation of 1-phenethyl-2-(4-hydroxyphenyl)indolizine

To a solution of 1-phenethyl-2-(4-methoxyphenyl)indolizine in acetic acid (12 mL) was added 48% HBr (40.6 mL) slowly. The mixture was stirred and heated at 100 °C for 8 hours. Solvent was removed in vacuo to afford the title compound, which was used without purification.

Step C Preparation of 1-phenethyl-2-[4-(3-piperidinylpropoxy)phenyl]-indolizine

To a solution of 1-phenethyl-2-(4-hydroxyphenyl)indolizine (0.33 mmol, 0.105 g) in 10 mL of N,N-dimethylformamide, was added sodium *tert*-butoxide (1.34 mmol, 0.129 g). The mixture was stirred and heated at 40 °C for 2

hours. 1-piperidinepropanyl chloride (0.33 mmol, 0.665 g) was added and the reaction mixture was heated at 60 °C for 16h. N,N-dimethylformamide was evaporated. Then water (40 mL) was added. After extraction with ethyl acetate (3 x 30 mL), dried over magnesium sulfate, evaporated to afford the title compound (0.085g, 59%). $^1$H NMR (400 MHz, $CDCl_3$) δ 7.85 (d, J = 6.9, 1 H), 7.38 (d, J = 8.7, 2H), 7.31 (s, 1 H), 7.28-7.24 (m, 3H), 7.20-7.14 (m, 3H), 6.97 (d, J = 8.7, 2H), 6.58 (ddd, J = 9.1, 6.5, 1.0, 1 H), 6.41 (t, J = 6.7, 1 H), 4.07 (t, J = 6.4, 2H), 3.16-3.12 (m, 2H), 2.86-2.82 (m, 2H), 2.56-2.46 (m, 6H), 2.07-2.00 ( M, 2H), 1.66-1.60 (m, 4H), 1.48-1.44 (m, 2H). $^{13}$C NMR (400 MHz, $CDCl_3$) δ 157.96, 142.6, 130.9, 130.00, 128.97, 128.65, 128.59, 128.47, 125.96, 124.97, 117.5, 116.1, 114.7, 110.28, 110.21, 110.18, 66.5, 56.2, 54.6, 37.9, 26.8, 26.6, 25.6, 24.3.

Example 12

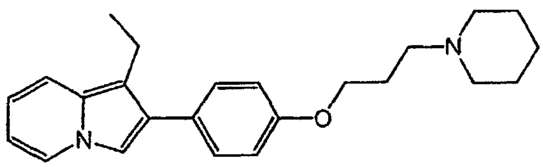

1-Ethyl-2-[4-(3-piperidinylpropoxy)phenyl]indolizine
Ki = 6 nM

Step A Preparation of 1-ethyl-2-(4-methoxyphenyl)indolizine

A solution of 2-bromo-4'-methoxyacetophenone (18 mmol, 4.16 g) and 2-propylpyridine (18 mmol, 2.45 g) in acetone (50 mL) was heated at reflux temperature for 8 hours. The quaternary salt precipitated out and was washed with cold acetone. The salt was collected and redissolved in hot water (50 mL). Potassium carbonate (54 mmol, 7.5 g) was added and the mixture was heated at 100 °C for 8 hours. After cooling to rt, solid was filtered and dried in vacuo to afford the title compound (3.4 g, 76%) as black solid, which was used without purification.

Step B Preparation of 1-ethyl-2-(4-hydroxyphenyl)indolizine

To a solution of 1-ethyl-2-(4-methoxyphenyl)indolizine (13.5 mmol, 3.4 g) in acetic acid (15 mL) was added 48% HBr (47 mL) slowly. The mixture was stirred and heated at 100 °C for 2 days. Solvent was removed in vacuo to give the title compound, which was used without purification.

Step C Preparation of 1-ethyl-2-[4-(3-piperidinylpropoxy)phenyl]indolizine

To a solution of 3-ethyl-2-(4-hydroxyphenyl)indolizine (0.44 mmol, 0.104 g) in 10 mL of N,N-dimethylformamide, was added sodium *tert*-butoxide (1.74 mmol, 0.168 g). The mixture was stirred and heated at 40 °C for 2 hours. 1-piperidinepropanyl chloride (0.44 mmol, 0.865 g) was added and the reaction mixture was heated at 60 °C for 16h. N,N-dimethylformamide was evaporated. Then water (40 mL) was added. After extraction with ethyl acetate (3 x 30 mL), dried over magnesium sulfate, evaporated to give the title compound (0.106g, 66%). $^1$H NMR (400 MHz, $CDCl_3$) δ 7.83 (dt, *J* = 7.0, 1.0, 1 H), 7.39 (d, *J* = 8.8, 2H), 7.33 (br d, 1 H), 7.28 (s, 1H), 6.95 (d, J = 8.8, 2H), 6.58 (ddd, J = 9.1, 6.4, 1.0, 1 H), 6.39 (t, *J* = 6.6, 1 H), 4.04 (t, *J* = 6.4, 2H), 2.85 (q, *J* = 7.5, 2H), 2.51 (m , 2H), 2.43 (br s, 4H), 2.03-1.98 (m, 2H), 1.64-1.58 (m, 4H), 1.51-1.45 (m, 2H), 1.19 (t, *J* = 7.5, 3H).

Example 13

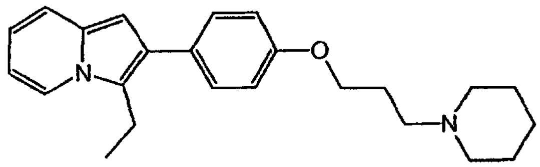

3-Ethyl-2-[4-(3-piperidinylpropoxy)phenyl]indolizine
Ki = 11 nM

Step A Preparation of 3-ethyl-2-(4-methoxyphenyl)indolizine

A solution of 2-bromo-2-ethyl-4'-methoxyacetophenone (7.78 mmol, 2.00 g) and 2-picoline (7.78 mmol, 0.77 mL) in acetone (40 mL) was heated at reflux temperature for 8 hours. The quaternary salt precipitated out and was washed with cold acetone. The salt was collected and redissolved in hot water (40 mL). Potassium carbonate (23 mmol, 3.23 g) was added and the mixture was heated at 100 °C for 8 hours. After cooling to rt, extracted with dichloromethane, dried over sodium sulfate, filtered, concentrate. Purification via silica gel chromatography in 5 % ethyl acetate/hexane to result the title compound (0.600g, 31%).

Step B Preparation of 3-ethyl-2-(4-hydroxyphenyl)indolizine

To a solution of 3-ethyl-2-(4-methoxyphenyl)indolizine in acetic acid (0.29 mL) was added 48% HBr (1.0 mL) slowly. The mixture was stirred and heated at 100 °C for 8 h. Solvent was removed in vacuo to give the title compound, which was used without purification.

Step C Preparation of 3-ethyl-2-[4-(3-piperidinylpropoxy)phenyl]indolizine

To a solution of 3-ethyl-2-(4-hydroxyphenyl)indolizine (0.29 mmol, 0.069 g) in 10 mL of N,N-dimethylformamide, was added sodium *tert*-butoxide (0.93 mmol, 0.089 g). The mixture was stirred and heated at 40 °C for 2 hours. 1-piperidinepropanyl chloride (0.29 mmol, 0.058 g) was added and the reaction mixture was heated at 60 °C for 6h. N,N-dimethylformamide was evaporated. Then water (40 mL) was added. After extraction with ethyl acetate (3 x 30 mL), dried over magnesium sulfate, and evaporated. Purification via silica gel chromatography in 25 % ethyl acetate/dichloromethane to result the title compound (0.013g, 12.4%).

Example 13 was synthesized using the same method as described in Example 4, and its inseparable by-product (B) (ratio of 13: B = 2:1) was submitted and tested as a mixture. However, a new synthetic method has been devised as described in Example 8. Wherein, we anticipate that Example 13 could be synthesized without the contamination of by-product (B).

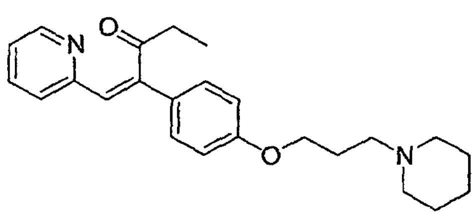

(B)

2-[4-(3-Piperidin-1-yl-propoxy)-phenyl]-1-pyridin-2-yl-pent-1-en-3-one

Example 14

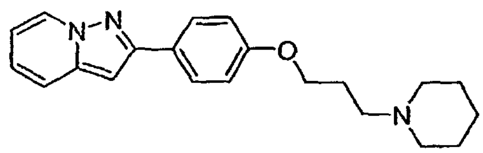

2-(4-Piperidylpropoxyphenyl)-7a-hydropyrazolo[1,5-a]pyridine
Ki = 12.5, MS ($MH^+$ 336)

Step A 1-Amino-2-methylpyridinium iodide

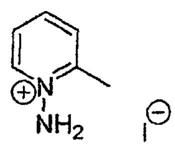

A mixture of 2-picoline (4.7 g) and hydroxylamine sulfonic acid (1.9 g) in water (15 mL) was heated at reflux temperature for 1 hour. The cooled reaction mixture was treated with $K_2CO_3$ (1.2 g) and diluted with absolute ethanol. The solution was then filtered through celite and the filtrated treated with 57% hydrogen iodide (4.2 mL). The reaction mixture was then cooled to -45 °C for 12 hours. The yellow precipitate was collected by filtration in a cold filter and washed with cold ether to yield the title compound (2.7 g).

Step B 1-[1-Aza-2-(p-methoxyphenyl)-1-enyl]-2-methylpyridium iodide

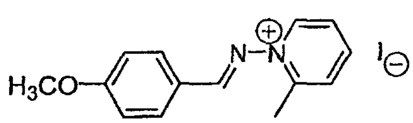

A mixture of the product of Step A (0.2 g), 4-methoxybenzylaldehyde (0.1 mL) and 4A molecular sieves in methanol (10 mL) was heated t reflux temperature for 20 hours. The reaction was cooled to ambient temperature and the excess solvent was removed under reduced pressure to yield a yellow solid containing the title compound that was used without further purification.

Step C 2-(4-Methoxyphenyl)-7a-hydropyrazolo[1,5-a]pyridine

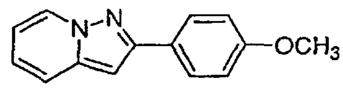

A mixture of the product from Step B (0.3 g), iodine (0.3 g) in pyridine (8 mL) was heated at reflux temperature for 6 hours. The reaction was cooled to ambient temperature, filtered through celite and the filtrate concentrated in vacuo. The residue was purified via silica gel chromatography (EtOAc/Hexanes) to give the title compound (0.02 g).

Step D 2-(4-Hydroxyphenyl)-7a-hydropyrazolo[1,5-a]pyridine

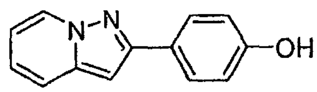

A mixture of the product from Step C (0.2 g) in acetic acid (1.0 mL) and hydrobromic acid (48%, 2.0 mL) was heated at 100 °C for 3 hours. It was then cooled to room temperature and concentrated in vacuo. The residue was triturated in ether and filtered to yield a yellow solid containing the title compound that was used without further purification (0.15).

Step E 2-(4-Piperidylpropoxyphenyl)-7a-hydropyrazolo[1,5-a]pyridine

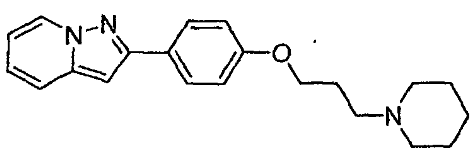

A mixture of the product of Step D (0.03 g) and sodium tert-butoxide (0.06 g) in DMA (5.0 mL) was stirred at room temperature for 30 min. 3-Chloropropyl-piperidine hydrochloride (0.05 g) was added and the reaction mixture was heated at 100 °C overnight. It was then cooled to room temperature, diluted with $H_2O$ and the resulting mixture was exacted with EtOAc (10 mL x 3). The combined organic layer was washed with $H_2O$ (15 mL x 5), dried over sodium sulfate, filtered and the filtrate was concentrated in vacuo. The residue was purified via silica gel chromatography (EtOAc/Hexanes) to give the title compound (0.02 g). $^1H$ NMR (400MHz, $CDCl_3$) δ 8.45 (dd, *J* = 7.0 and 0.7 Hz, 1H), 7.88 (dd, *J* = 6.9 and 1.8 Hz, 2H), 7.47 (d, *J* = 8.9 Hz, 1 H), 7.07 (t, *J* = 0.9 Hz, 1H), 6.98 (d, *J* = 6.8 Hz, 2H), 6.70 (m, 2H), 4.06 (t, *J* = 6.4 Hz, 2H), 2.50 (t, *J* = 7.3 Hz, 2H), 2.42 (,m, 4H), 2.01 (p, *J* = 7.3 Hz, 2H), 1.60 (m, 4H), 1.45 (m, 2H).

F. Biological Examples

Biological Example 1

1(A) Transfection of cells with human histamine receptor

A 10 cm tissue culture dish with a confluent monolayer of SK-N-MC cells was split two days prior to transfection. Using sterile technique the media was removed and the cells were detached from the dish by the addition of

trypsin. One fifth of the cells were then placed onto a new 10cm dish. Cells were grown in a 37°C incubator with 5% $CO_2$ in Minimal Essential Media Eagle with 10% Fetal Bovine Serum. After two days cells were approximately 80% confluent. These were removed from the dish with trypsin and pelleted in a clinical centrifuge. The pellet was then re-suspended in 400 µL complete media and transferred to an electroporation cuvette with a 0.4 cm gap between the electrodes (Bio-Rad #165-2088). One microgram supercoiled $H_3$ receptor cDNA was added to the cells and mixed. The voltage for the electroporation was set at 0.25 kV, the capacitance is set at 960 µF.

After electroporation the cells were diluted into 10 mL complete media and plated onto four 10 cm dishes. Due to the variability in the efficiency of electroporation, four different concentrations of cells were plated. The ratios used were: 1:20, 1:10, and 1:5, with the remainder of the cells being added to the fourth dish. The cells were allowed to recover for 24 hours before adding the selection media (complete media with 600 µg/ml G418). After 10 days dishes were analyzed for surviving colonies of cells. Dishes with well-isolated colonies were used. Cells from individual colonies were isolated and tested. SK-N-MC cells were used because they give efficient coupling for inhibition of adenylate cyclase. The clones that gave the most robust inhibition of adenylate cyclase in response to histamine were used for further study.

1(B) [3H]-N-methylhistamine binding

Cell pellets from histamine $H_3$ receptor-expressing SK-N-MC cells were homogenized in 20mM TrisHCl/ 0.5mM EDTA. Supernatants from a 800 g spin were collected, reccentrifuged at 30,000 g for 30 minutes. Pellets were rehomogenized in 50 mM Tris/5mM EDTA (pH 7.4). Membranes were incubated with 0.8 nM [$^3$H]-N-methylhistamine plus/minus test compounds for 45 minutes at 25°C and harvested by rapid filtration over GF/C glass fiber filters (pretreated with 0.3% polyethylenimine) followed by four washes with ice cold buffer. Filters were dried, added to 4 mL scintillation cocktail and then counted on a liquid scintillation counter. Non-specific binding was defined with 10 µM histamine. $Pk_I$ values were calculated based on a $K_D$ of 800 pM and a ligand concentration ([L]) of 800 pM according to the formula:

$$K_I=(IC_{50})/(1 + ([L]/(K_D))$$

F. Other Embodiments

The features and advantages of the invention are apparent to one of ordinary skill in the art. Based on this disclosure, including the summary, detailed description, background, examples, and claims, one of ordinary skill in the art will be able to make modifications and adaptations to various conditions and usages.

**Claims**

**1.** A compound of formula (I)(C):

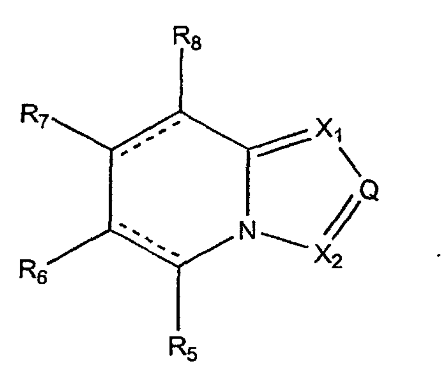

wherein
the dashed lines are both present to form two carbon-carbon double bonds; or are both absent;
$X_1$ is $CR_1$, wherein $R_1$ is H, C $_{1-6}$ alkyl, phenyl, cyano, or phenyl(C $_{1-6}$ alkyl);

one of Q and $X_2$ is C-Ar and the other is $CR_3$ or N, where $R_3$ is H or $C_{1-6}$ alkyl;
Ar is:

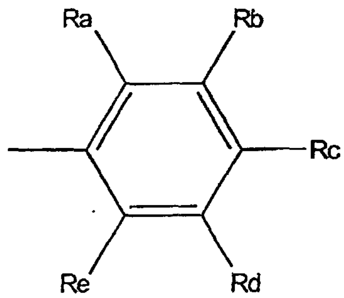

each of $R_5$, $R_6$, $R_7$, and $R_8$ are independently selected from H, $C_{1-3}$ alkyl, halo, and $C_{1-3}$ alkoxy;

one of $R_a$, $R_b$, $R_c$, $R_d$, and $R_e$ is WZ, and the others are independently selected from H, $C_{1-6}$ alkyl, halo, and $C_{1-6}$ alkoxy;

W is -O-, $C_{1-6}$ alkoxy, or $C_{1-6}$ alkylamino;

Z is $C_{2-8}$ heterocyclic radical, optionally including in the ring up to 3 additional heteroatoms or moieties independently selected from O, N, NH, S, SO, and $SO_2$ with at least one basic N; or Z is $NR_{11}R_{12}$ where each of $R_{11}$ and $R_{12}$ is independently selected from H, $C_{1-6}$ alkyl, phenyl, benzyl, $C_{3-8}$ cycloalkyl, and $C_{2-5}$ heterocyclic radical; or $NR_{11}R_{12}$ taken together is $C_{6-8}$ cycloalkylimino radical;

each of the above hydrocarbyl, heteroalkyl, or heterocyclic groups being optionally substituted with between 1 and 3 substituents selected from

$C_{1-3}$ alkyl, halo, hydroxy, phenyl, and phenyl($C_{1-3}$ alkyl); and wherein each of the above heterocyclic groups may be attached to the rest of the molecule by a carbon atom or a heteroatom; or a pharmaceutically acceptable salt, amide, ester, or hydrate thereof.

**2.** A compound of claim 1, wherein one of $R_a$, $R_b$, $R_c$, $R_d$, and $R_e$ is WZ, and the others are independently selected from H, $C_{1-3}$ alkyl, fluoro, chloro, and $C_{1-3}$ alkoxy.

**3.** A compound of claim 1, wherein between 1 and 2 of $R_1$, $R_3$, $R_5$, $R_6$, $R_7$, and $R_8$ independently comprise methyl, ethyl, $-CH_2-$, $-CH_2-CH_2-$, or phenyl.

**4.** A compound of claim 3, wherein one of $R_1$ and $R_3$ comprises methyl, ethyl, $-CH_2-$, $-CH_2-CH_2-$, or phenyl.

**5.** A compound of claim 1, wherein the dashed lines are absent.

**6.** A compound of claim 1, wherein the dashed lines are carbon-carbon double bonds.

**7.** A compound of claim 1, wherein Q is C-Ar and $X_2$ is $CR_3$ or N, where $R_3$ is H or $C_{1-4}$ alkyl.

**8.** A compound of claim 1, wherein at least two of the following apply: Z is N-piperidyl; W comprises propoxy or (N-methyl)propylamino; and three of $R_a$, $R_b$, $R_d$, and $R_e$ are each H.

**9.** A compound of claim 1, wherein WZ is 3-(N-piperidyl)propoxy or 3-(N-piperidyl)-(N-methyl)propylamino.

**10.** A compound of claim 1, wherein $X_2$ is $CR_3$.

**11.** A compound of claim 1, wherein
$R_1$ is H, methyl, ethyl, propyl, butyl, phenyl, benzyl, or phenethyl;
$R_3$ is H or $C_{1-2}$ alkyl;
each of $R_5$, $R_6$, $R_7$, $R_8$ and $R_e$ is independently H or methyl; and one of $R_a$, $R_b$, $R_c$ and $R_d$ is WZ; and the remaining three are each H; W is $C_{2-4}$ alkoxy or $C_{2-4}$ alkylamino; and Z is $C_{2-8}$ heterocyclic radical, optionally including in the ring up to 3 additional heteroatoms or moieties independently selected from O, N, NH, S, SO, and $SO_2$ with at least one basic N; or Z is $NR_{11}R_{12}$ where each of $R_{11}$ and $R_{12}$ is independently selected from H, $C_{1-6}$ alkyl,

phenyl, benzyl, C $_{3-8}$ cycloalkyl, and C $_{2-5}$ heterocyclic radical; or $NR_{11}R_{12}$ taken together is C $_{6-8}$ cycloalkylimino radical.

**12.** A compound of claim 1, selected from 3-Methyl-2-(4-piperidinylpropoxyphenyl)indolizine; 1-Methyl-2-(2-methyl-4-(3-piperidinylpropoxyphenyl))indolizine; 1-Phenyl-2-[4-(3-piperidinylpropoxy)phenyl]indolizine; 1-Phenethyl-2-[4-(3-piperidinylpropoxy)phenyl]indolizine; 1-Ethyl-2-[4-(3-piperidinylpropoxy)phenyl]indolizine; 1-Methyl-2-[4-(3-piperidinylpropoxy)-phenyl]-indolizine; 2-(3-Piperidinopropoxyphenyl)indolizine and 3-Ethyl-2-[4-(3-piperidinylpropoxy)phenyl]indolizine. [5-Methyl-2-(4-piperidinylpropoxyphenyl)indolizine; and 8-Methyl-2-[4-(3-piperidinylpropoxy)-phenyl]-indolizine].

**13.** A compound of claim 1, having the formula 2-(4-Piperidylpropoxyphenyl)-7a-hydropyrazoio[1,5-2]pyridine.

**14.** A pharmaceutical composition comprising a compound of formula (I)(C) and a pharmaceutically-acceptable carrier.

**15.** A pharmaceutical composition of claim 14, wherein said compound is a compound of formula (I)(C), wherein
$R_1$ is H, methyl, ethyl, propyl, butyl, phenyl, benzyl, or phenethyl;
$R_3$ is H or C $_{1-2}$ alkyl;
each of $R_5$, $R_6$, $R_7$, $R_8$ and $R_e$ is independently H or methyl; and
one of $R_a$, $R_b$, $R_c$ and $R_d$ is WZ; and the remaining three are each H; W is C $_{2-4}$ alkoxy or C $_{2-4}$ alkylamino; and Z is C $_{2-8}$ heterocyclic radical, optionally including in the ring up to 3 additional heteroatoms or moieties independently selected from O, N, NH, S, SO, and $SO_2$ with at least one basic N; or Z is $NR_{11}R_{12}$ where each of $R_{11}$ and $R_{12}$ is independently selected from H, C $_{1-6}$ alkyl, phenyl, benzyl, C $_{3-8}$ cycloalkyl, and C $_{2-5}$ heterocyclic radical; or $NR_{11}R_{12}$ taken together is C $_{6-8}$ cycloalkylimino radical.

**16.** A pharmaceutical composition of claim 15, wherein said compound has a formula selected from 3-Methyl-2-(4-piperidinylpropoxyphenyl)indolizine; 1-Methyl-2-(2-methyl-4-(3-piperidinylpropoxyphenyl))indolizine; 1-Phenyl-2-[4-(3-piperidinylpropoxy)phenyl]indolizine; 1-Phenethyl-2-[4-(3-piperidinylpropoxy)phenyl]indolizine; 1-Ethyl-2-[4-(3-piperidinylpropoxy)phenyl]indolizine; 1-Methyl-2-[4-(3-piperidinylpropoxy)-phenyl]-indolizine; 2-(3-Piperidinopropoxyphenyl)indolizine and 3-Ethyl-2-[4-(3-piperidinylpropoxy)phenyl]indolizine. [5-Methyl-2-(4-piperidinylpropoxyphenyl)indolizine; 8-Methyl-2-[4-(3-piperidinylpropoxy)-phenyl]-indolizine; and 2-(4-Piperidylpropoxyphenyl)-7a-hydropyrazolo [1, 5-2] pyridine.

**17.** The use of a compound according to any of claims 1 to 13 in the manufacture of a medicament for treating disorders mediated by the histamine $H_3$ receptor in a patient.

**18.** The use of a compound according to any of claims 1 to 13 in the manufacture of a medicament for treating a patient with a central nervous system disorder.

**19.** The use according to claim 18, wherein said central nervous system disorder is selected from sleep/wake disorders, arousal/vigilance disorders, dementia, Alzheimer's disease, epilepsy, narcolepsy, eating disorders, motion sickness, vertigo, attention deficit hyperactivity disorder, learning and memory disorders, mild cognitive impairment, and schizophrenia.

**20.** The use according to claim 18, wherein said disorder is selected from sleep/wake disorders, arousal/vigilance disorders, mild cognitive impairment, attention deficit hyperactivity disorder, and learning and memory disorders.

**21.** The use of a compound according to any of claims 1 to 13 in the manufacture of a medicament for treating a patient with an upper airway allergic response.

**Patentansprüche**

**1.** Verbindung der Formel (I)(C):

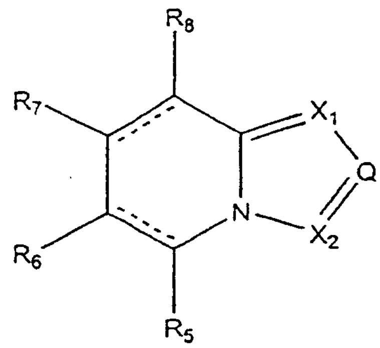

wobei
die gestrichelten Linien beide vorhanden sind, um zwei Kohlenstoff-Kohlenstoff-Doppelbindungen zu bilden; oder beide abwesend sind;
$X_1$ $CR_1$ ist, wobei $R_1$ H, $C_{1-6}$-Alkyl, Phenyl, Cyano, oder Phenyl ($C_{1-6}$-Alkyl) ist;
eines von Q und $X_2$ C-Ar ist und das andere $CR_3$ oder N ist, wobei $R_3$ H oder $C_{1-6}$-Alkyl ist;
Ar ist:

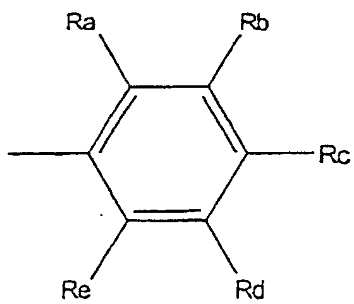

wobei jedes von $R_5$, $R_6$, $R_7$ und $R_8$ unabhängig voneinander ausgewählt sind aus H, $C_{1-3}$-Alkyl, Halogen, und $C_{1-6}$-Alkoxy;
eines von $R_a$, $R_b$, $R_c$, $R_d$ und $R_e$ WZ ist, und die anderen unabhängig voneinander ausgewählt sind aus H, $C_{1-6}$-Alkyl, Halogen und $C_{1-6}$-Alkoxy;
W -O-, $C_{1-6}$-Alkoxy oder $C_{1-6}$-Alkylamino ist;
Z ein heterozyklisches $C_{2-8}$-Radikal ist, das fakultativ in dem Ring bis zu 3 zusätzliche Heteroatome oder Gruppen einschließt, die unabhängig voneinander ausgewählt sind aus O, N, NH, S, SO, und $SO_2$, mit wenigstens einem basischen N; oder Z $NR_{11}R_{12}$ ist, wobei jedes aus $R_{11}$ und $R_{12}$ unabhängig voneinander ausgewählt ist aus H, $C_{1-6}$-Alkyl, Phenyl, Benzyl, $C_{3-8}$-Cycloalkyl und heterozyklisches $C_{2-5}$-Radikal; oder $NR_{11}R_{12}$ zusammengenommen ein $C_{6-8}$-Cycloalkyliminoradikal ist;
jede der obigen Kohlenwasserstoff-, Heteroalkyl- oder heterozyklischen Gruppen fakultativ mit zwischen 1 und 3 Substituenten substituiert ist, ausgewählt aus $C_{1-3}$-Alkyl, Halogen, Hydroxy, Phenyl und Phenyl($C_{1-3}$-Alkyl); und wobei jede der obigen heterozyklischen Gruppen an den Rest des Moleküls durch ein Kohlenstoffatom oder ein Heteroatom angehängt sein kann;
oder ein pharmazeutisch annehmbares Salz, Amid oder Ester oder Hydrat davon.

**2.** Verbindung nach Anspruch 1, wobei eines von $R_a$, $R_b$, $R_c$, $R_d$, und $R_e$ WZ ist, und die anderen unabhängig ausgewählt sind aus H, $C_{1-3}$-Alkyl, Fluor, Chlor und $C_{1-3}$-Alkoxy.

**3.** Verbindung nach Anspruch 1, wobei zwischen 1 und 2 von $R_1$, $R_3$, $R_5$, $R_6$, $R_7$ und $R_8$ unabhängig voneinander Methyl, Ethyl, $-CH_2-$, $-CH_2-CH_2-$, oder Phenyl umfassen.

**4.** Verbindung nach Anspruch 3, wobei eines von $R_1$ und $R_3$ Methyl, Ethyl, $-CH_2-$, $-CH_2-CH_2-$ oder Phenyl umfasst.

**5.** Verbindung nach Anspruch 1, wobei die gestrichelten Linien abwesend sind.

**6.** Verbindung nach Anspruch 1, wobei die gestrichelten Linien Kohlenstoff-Kohlenstoff-Doppelbindungen sind.

**7.** Verbindung nach Anspruch 1, wobei Q C-Ar ist und $X_2$ $CR_3$ oder N ist, wobei $R_3$ H oder $C_{1-4}$-Alkyl ist.

**8.** Verbindung nach Anspruch 1, wobei wenigstens zwei der folgenden zutreffen: Z ist N-Piperidyl; W umfaßt Propoxy oder (N-Methyl)-propylamino; und drei von $R_a$, $R_b$, $R_d$, und $R_e$ sind jeweils H.

**9.** Verbindung nach Anspruch 1, wobei WZ 3-(N-Piperidyl)propoxy oder 3-(N-Piperidyl)-(N-methyl)propylamino ist.

**10.** Verbindung nach Anspruch 1, wobei $X_2$ $CR_3$ ist.

**11.** Verbindung nach Anspruch 1, wobei $R_1$ H, Methyl, Ethyl, Propyl, Butyl, Phenyl, Benzyl oder Phenethyl ist;
$R_3$ H oder $C_{1-2}$-Alkyl ist;
jedes von $R_5$, $R_6$, $R_7$, $R_8$ und $R_e$ unabhängig voneinander H oder Methyl sind; und eines von $R_a$, $R_b$, $R_c$, und $R_d$ WZ ist; und die verbleibenden drei jeweils H sind;
W $C_{2-4}$-Alkoxy oder $C_{2-4}$-Alkylamino ist; und Z ein heterozyklisches $C_{2-8}$-Radikal ist, das fakultativ im Ring bis zu drei zusätzliche Heteroatome oder Gruppen einschließt, die unabhängig voneinander ausgewählt sind aus O, N, NH, S, SO und $SO_2$ mit wenigstens einem basischen N; oder Z $NR_{11}R_{12}$ ist, wobei jedes von $R_{11}$ und $R_{12}$ unabhängig voneinander ausgewählt ist aus H, $C_{1-6}$-Alkyl, Phenyl, Benzyl, $C_{3-8}$-Cycloalkyl und ein heterozyklisches $C_{2-5}$-Radikal; oder $NR_{11}R_{12}$ zusammengenommen ein $C_{6-8}$-Cycloalkyliminoradikal ist.

**12.** Verbindung nach Anspruch 1, ausgewählt aus 3-Methyl-2-(4-piperidinylpropoxyphenyl)indolizin; 1-Methyl-2-(2-methyl-4-(3-piperidinylpropoxyphenyl))indolizin; 1-Phenyl-2-[4-(3-piperidinylpropoxy)phenyl]indolizin; 1-Phenethyl-2-[4-(3-piperidinylpropoxy)phenyl]indolizin; 1-Ethyl-2-[4-(3-piperidinylpropoxy)phenyl] indolizin; 1-Methyl-2-[4-(3-piperidinylpropoxy)-phenyl]-indolizin; 2-(3-Piperidinopropoxyphenyl)indolizin und 3-Ethyl-2-[4-(3-piperidinylpropoxy)phenyl]indolizin;[5-Methyl-2-(4-piperidinylpropoxyphenyl)indolizin;und8-Methyl-2-[4-(3-piperidinylpropoxy)-phenyl]-indolizin].

**13.** Verbindung nach Anspruch 1 mit der Formel 2-(4-Piperidylpropoxyphenyl)-7ahydropyrazolo[ 1,5-2]pyridin.

**14.** Eine pharmazeutische Zusammensetzung, umfassend eine Verbindung der Formel (I)(C) und einen pharmazeutisch annehmbaren Träger.

**15.** Pharmazeutische Zusammensetzung nach Anspruch 14, wobei besagte Verbindung eine Verbindung der Formel (I)(C) ist, wobei $R_1$ H, Methyl, Ethyl, Propyl, Butyl, Phenyl, Benzyl oder Phenethyl ist; $R_3$ H oder $C_{1-2}$-Alkyl ist; jedes von $R_5$, $R_6$, $R_7$, $R_8$ und $R_e$ unabhängig H oder Methyl ist; und eines von $R_a$, $R_b$, $R_c$ und $R_d$ WZ ist, und die verbleibenden drei jeweils H sind; W $C_{2-4}$-Alkoxy oder $C_{2-4}$-Alkylamino ist; und Z ein heterozyklisches $C_{2-8}$-Radikal ist, das fakultativ im Ring bis zu drei zusätzliche Heteroatome oder Gruppen einschließt, unabhängig voneinander ausgewählt aus O, N, NH, S, SO und $SO_2$, mit wenigstens einem basischen N; oder Z $NR_{11}R_{12}$ ist, wobei jedes von $R_{11}$ und $R_{12}$ unabhängig voneinander ausgewählt ist aus H, $C_{1-6}$-Alkyl, Phenyl, Benzyl, $C_{3-8}$-Cycloalkyl und ein heterozyklisches $C_{2-5}$-Radikal; oder $NR_{11}R_{12}$ zusammengenommen $C_{6-8}$-Cycloalkyliminoradikal ist.

**16.** Pharmazeutische Zusammensetzung nach Anspruch 15, wobei die Verbindung eine Formel hat, ausgewählt aus 3-Methyl-2-(4-piperidinylpropoxyphenyl)indolizin; 1-Methyl-2-(2-methyl-4-(3-piperidinylpropoxyphenyl))indolizin; 1-Phenyl-2-[4-(3-piperidinylpropoxy)phenyl]indolizin; 1-Phenethyl-2-[4-(3-piperidinylpropoxy)phenyl]indolizin; 1-Ethyl-2-[4-(3-piperidinylpropoxy)phenyl]indolizin; 1-Methyl-2-[4-(3-piperidinylpropoxy) -phenyl]-indolizin; 2-(3-Piperidinopropoxyphenyl)indolizin und 3-Ethyl-2-[4-(3-piperidinylpropoxy)phenyl]indolizin; [5-Methyl-2-(4-piperidinylpropoxyphenyl)indolizin; 8-Methyl-2-[4-(3-piperidinylpropoxy)-phenyl]-indolizin; und 2-(4-Piperidylpropoxyphenyl)-7a-hydropyrazolo[ 1,5-2]pyridin.

**17.** Verwendung einer Verbindung nach einem der Ansprüche 1 bis 13 zur Herstellung eines Medikaments zur Behandlung von Störungen bei einem Patienten, die durch den Histamine-$H_3$-Rezeptor vermittelt werden.

**18.** Verwendung einer Verbindung nach einem der Ansprüche 1 bis 13 zur Herstellung eines Medikaments zur Behandlung eines Patienten mit einer Krankheit des zentralen Nervensystems.

**19.** Verwendung nach Anspruch 18, wobei die Krankheit des zentralen Nervensystems ausgewählt ist aus Schlaf/ Wach-Störungen, Aufwach/Vigilanz-Störungen, Demenz, Alzheimer, Epilepsie, Narkolepsie, Eßstörungen, Bewegungskrankheit, Vertigo, Aufmerksamkeitsdefizit-Hyperaktivität-Störung, Lern- und Gedächtnisstörungen, milde kognitive Beeinträchtigung und Schizophrenie.

**20.** Verwendung nach Anspruch 18, wobei die Störung ausgewählt ist aus Schlaf/Wach-Störungen, Aufwach/Vigilanz-Störungen, milde kognitive Beeinträchtigung, Aufmerksamkeitsdefizit-Hyperaktivität-Störung und Lern- und Gedächtnisstörungen.

**21.** Verwendung einer Verbindung nach einem der Ansprüche 1 bis 13 zur Herstellung eines Medikaments zum Behandeln eines Patienten mit einer allergischen Reaktion der oberen Atemwege.

## Revendications

**1.** Composé de formule (I) (C) :

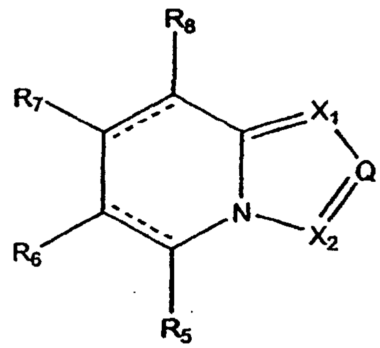

dans laquelle
les tirets sont présents tous les deux pour former des doubles liaisons carbone - carbone ou sont tous les deux absents ;
$X_1$ est $CR_1$ dans lequel $R_1$ est un groupement alkyle $C_{1-6}$, phényle, cyano ou phényle (alkyle $C_1$-6) ;
l'un des Q et $X_2$ est C-Ar et l' autre est $CR_3$ ou N, dans lequel $R_3$ est H ou un groupement alkyle $C_{1-6}$ ;
Ar est :

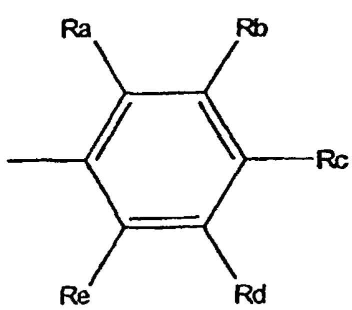

chacun des $R_5$, $R_6$, $R_7$ et $R_8$ sont indépendamment choisis parmi H, un groupement alkyle $C_{1-3}$, un halogène et un groupement alcoxy $C_{1-3}$ ;
l'un des $R_a$, $R_b$, $R_c$, $R_d$ et $R_e$ est WZ, et les autres sont indépendamment choisis parmi H, un groupement alkyle $C_{1-6}$, un halogène et un groupement alcoxy $C_{1-6}$ ;
W est -O-, un groupement alcoxy $C_{1-6}$ ou alkylamino $C_{1-6}$ ;
Z est un radical hétérocyclique $C_{2-8}$, comprenant facultativement dans le cycle jusqu'à 3 hétéroatomes ou fragments supplémentaires indépendamment choisis parmi O, N, NH, S, SO et $SO_2$ avec au moins un N basique ; ou Z est $NR_{11}R_{12}$ dans lequel chacun des $R_{11}$ et $R_{12}$ est indépendamment choisi parmi H, un groupement alkyle $C_{1-6}$, phényle, benzyle, cycloalkyle $C_{3-8}$ et un radical hétérocycloalkyle $C_{2-5}$ ; ou $NR_{11}R_{12}$ pris ensemble est un radical cycloalkylimino $C_{6-8}$ ;
chacun des groupements hydrocarbonyle, hétéroalkyle ou hétérocyclique précédents étant facultativement subs-

tués avec entre 1 et 3 substituants choisis parmi
un groupement alkyle $C_{1-3}$, un halogène, un hydroxy, un phényle, et un phényle (alkyle $C_{1-3}$) ; et dans lequel chacun des groupements hétérocycliques précédents peut être attaché au reste de la molécule par un atome de carbone ou un hétéroatome ; ou un sel, une amide, un ester ou un hydrate pharmaceutiquement acceptable de celui-ci.

**2.** Composé selon la revendication 1, dans lequel l'un de $R_a$, $R_b$, $R_c$, $R_d$ et $R_e$ est WZ, et les autres sont indépendamment choisis parmi H, un groupement alkyle $C_{1-3}$, un fluor, un chlore et un groupement alcoxy $C_{1-3}$.

**3.** Composé selon la revendication 1, dans lequel entre 1 et 2 des $R_1$, $R_3$, $R_5$, $R_6$, $R_7$ et $R_8$ comprennent indépendamment un méthyle, un éthyle, $-CH_2-$, $-CH_2-CH_2-$ ou un phényle.

**4.** Composé selon la revendication 3, dans lequel l'un des $R_1$ et $R_3$ comprend un méthyle, un éthyle, $-CH_2-$, $-CH_2-CH_2-$ ou un phényle.

**5.** Composé selon la revendication 1, dans lequel les tirets sont absents.

**6.** Composé selon la revendication 1, dans lequel les tirets sont des doubles liaisons carbone - carbone.

**7.** Composé selon la revendication 1, dans lequel Q est C-Ar et $X_2$ est $CR_3$ ou N, dans lequel $R_3$ est H ou un groupement alkyle $C_{1-4}$.

**8.** Composé selon la revendication 1, dans lequel au moins deux des propositions suivantes s'appliquent : Z est un N-pipéridyle ; W comprend un groupement propoxy ou (N-méthyl) propylamino et trois des $R_a$, $R_b$, $R_d$ et $R_e$ sont chacun H.

**9.** Composé selon la revendication 1, dans lequel WZ est le groupement 3-(N-pipéridyl) propoxy ou 3-(N-pipéridyl) -(N-méthyl) propylamino.

**10.** Composé selon la revendication 1, dans lequel $X_2$ est $CR_3$.

**11.** Composé selon la revendication 1, dans lequel
$R_1$ est H, un méthyle, un éthyle, un propyle, un butyle, un phényle, un benzyle ou un phénéthyle ;
$R_3$ est H ou un groupement alkyle $C_{1-2}$ ;
chacun des $R_5$, $R_6$, $R_7$, $R_8$ et $R_e$ est indépendamment H ou un méthyle ; et l'un des $R_a$, $R_b$, $R_c$ et $R_d$ est WZ ; et les trois restant sont chacun H ; W est un groupement alcoxy $C_{2-4}$ ou alkylamino $C_{2-4}$ ; et Z est un radical hétérocyclique $C_{2-8}$, comprenant facultativement dans le cycle jusqu'à 3 hétéroatomes ou fragments supplémentaires choisis indépendamment parmi 0, N, NH, S, SO et $SO_2$ avec au moins un N basique ; ou Z est $NR_{11}R_{12}$ ou chacun des $R_{11}$ et $R_{12}$ est indépendamment choisi parmi H, un radical alkyle $C_{1-6}$, phényle, benzyle, cycloalkyle $C_{3-8}$ et hétérocyclique $C_{2-5}$ ; ou $NR_{11}R_{12}$ pris ensemble est un radical cycloalkylimino $C_{6-8}$.

**12.** Composé selon la revendication 1, choisi parmi le 3-méthyl-2-(4-pipéridinylpropoxyphényl) indolizine ; le 1-méthyl-2-(2-méthyl-4-(3-pipéridinylpropoxyphényl)) indolizine ; le 1-phényl-2-[4-(3-pipéridinylpropoxy) phényl] indolizine ; le 1-phénéthyl-2-[4-(3-pipéridinylpropoxy) phényl] indolizine ; le 1-éthyl-2-[4-(3-pipéridinylpropoxy) phényl] indolizine ; le 1-méthyl-2-[4-(3-pipéridinylpropoxy) phényl] indolizine ; le 2-(3-pipéridinopropoxyphényl) indolizine et le 3-éthyl-2-[4-(3-pipéridinylpropoxy) phényl] indolizine ; le 5-méthyl-2-(4-pipéridinylpropoxyphényl) indolizine et le 8-méthyl-2-[4-(3-pipéridinylpropoxy) phényl] indolizine.

**13.** Composé selon la revendication 1, ayant la formule 2-(4-pipéridylpropyloxyphényl)-7a-hydropyrazolo [1,5-2] pyridine.

**14.** Composition pharmaceutique comprenant un composé de formule (I) (C) et un vecteur pharmaceutiquement acceptable.

**15.** Composition pharmaceutique selon la revendication 14, dans laquelle ledit composé est un composé de formule (I) (C), dans laquelle $R_1$ est H, un méthyle, un éthyle, un propyle, un butyle, un phényle, un benzyle ou un phénéthyle ;
$R_3$ est H ou un groupement alkyle $C_{1-2}$ ;

chacun des $R_5$, $R_6$, $R_7$, $R_8$ et $R_e$ est indépendamment H ou un méthyle ; et l'un des $R_a$, $R_b$, $R_c$ et $R_d$ est WZ ; et les trois restant sont chacun H ; W est un groupement alcoxy $C_{2-4}$ ou alkylamino $C_{2-4}$ ; et Z est un radical hétérocyclique $C_{2-8}$, comprenant facultativement dans le cycle jusqu'à 3 hétéroatomes ou fragments supplémentaires choisis indépendamment parmi O, N, NH, S, SO et $SO_2$ avec au moins un N basique ; ou Z est $NR_{11}R_{12}$ ou chacun des $R_{11}$ et $R_{12}$ est indépendamment choisi parmi H, un radical alkyle $C_{1-6}$, phényle, benzyle, cycloalkyle $C_{3-8}$ et hétérocyclique $C_{2-5}$ ; ou $NR_{11}R_{12}$ pris ensemble est un radical cycloalkyl imino $C_{6-8}$.

**16.** Composition pharmaceutique selon la revendication 15, dans laquelle ledit composé a une formule choisie parmi le 3-méthyl-2-(4-pipéridinylpropoxyphényl) indolizine ; le 1-méthyl-2-(2-méthyl-4-(3-pipéridinylpropoxyphényl)) indolizine ; le 1-phényl-2-[4-(3-pipéridinylpropoxy) phényl] indolizine ; le 1-phénéthyl-2-[4-(3-pipéridinylpropoxy) phényl] indolizine ; le 1-éthyl-2-[4-(3-pipéridinylpropoxy) phényl] indolizine ; le 1-méthyl-2-[4-(3-pipéridinylpropoxy) phényl] indolizine ; le 2-(3-pipéridinopropoxyphényl) indolizine et le 3-éthyl-2-[4-(3-pipéridinylpropoxy) phényl] indolizine ; le 5-méthyl-2-(4-pipéridinylpropoxyphényl) indolizine ; le 8-méthyl-2-[4-(3-pipéridinylpropoxy) phényl] indolizine et la 2-(4-pipéridylpropoxyphényl)-7a-hydropyrazolo [1,5-2] pyridine. ,

**17.** Utilisation d'un composé selon l'une quelconque des revendications 1 à 13, dans la fabrication d'un médicament pour traiter des troubles à médiation par le récepteur de l'histamine $H_3$ chez un patient.

**18.** Utilisation d'un composé selon l'une quelconque des revendications 1 à 13, dans la fabrication d'un médicament pour traiter un patient avec un trouble du système nerveux central.

**19.** Utilisation selon la revendication 18, dans laquelle le trouble du système nerveux central est choisi parmi les troubles du sommeil/éveil, les troubles du réveil/vigilance, la démence, la maladie d'Alzheimer, l'épilepsie, la narcolepsie, les troubles de l'alimentation, le mal des transports, le vertige, le trouble de l'hyperactivité avec déficit d'attention, les troubles de l'apprentissage et de la mémoire, le trouble cognitif modéré et la schizophrénie.

**20.** Utilisation selon la revendication 18, dans laquelle ledit trouble est choisi parmi les troubles du sommeil/éveil, les troubles du réveil/vigilance, le trouble cognitif modéré, le trouble de l'hyperactivité avec déficit d'attention et les troubles de l'apprentissage et de la mémoire.

**21.** Utilisation d'un composé selon l'une quelconque des revendications 1 à 13, dans la fabrication d'un médicament pour traiter un patient avec une réponse allergique des voies aériennes supérieures.